# EUROPEAN PATENT SPECIFICATION

(11) **EP 2 496 183 B1**
(45) Date of publication and mention of the grant of the patent: **02.09.2015**
(21) Application number: 10829105.5
(22) Date of filing: 04.11.2010
(51) Int. Cl.: A61F 2/38

(54) **PATIENT-ADAPTED AND IMPROVED ORTHOPEDIC IMPLANTS, DESIGNS AND RELATED TOOLS**
PATIENTENADAPTIERTE UND VERBESSERTE ORTHOPÄDISCHE IMPLANTATE, ENTWÜRFE UND ZUGEHÖRIGE INSTRUMENTE
IMPLANTS ORTHOPÉDIQUES AMÉLIORÉS ET ADAPTÉS AU PATIENT, MODÈLES ET OUTILS ASSOCIÉS

(30) Priority: 04.11.2009 US 280493 P; 18.12.2009 US 284458 P; 09.03.2010 US 339766 P
(43) Date of publication of application: 12.09.2012
(73) Proprietor: ConforMIS, Inc., Bedford, MA 01730 (US)
(72) Inventor: BOJARSKI, Raymond, A., Attleboro, MA 02703 (US); LANG, Philipp, Lexington, MA 02421 (US); CHAO, Nam, Marlborough, MA 01752 (US); FITZ, Wolfgang, Sherborn, MA 01770 (US); SLAMIN, John, Wrentham, MA 02093 (US); STEINES, Daniel, Lexington, MA 02421 (US); MINAS, Thomas, Dover MA 02030 (US)
(74) Representative: Grund, Martin
(86) International application number: PCT/US2010/055483
(87) International publication number: WO 2011/056995

(56) References cited:
- WO-A1-2005/051240
- WO-A2-2010/099231
- US-A1- 2007 100 462
- US-A1- 2007 118 055
- US-A1- 2007 276 501
- US-A1- 2008 172 125
- US-A1- 2008 195 108
- US-A1- 2008 215 059
- US-A1- 2008 262 624
- US-A1- 2008 281 329

## Description

### TECHNICAL FIELD

The invention relates to patient-specific orthopedic implants.

### BACKGROUND

Generally, a diseased, injured or defective joint, such as, for example, a joint exhibiting osteoarthritis, has been repaired using standard off-the-shelf implants and other surgical devices. Only recently has the concept of patient-specific implants tailored to an individual patient's joint become available. Such patient-specific implants, such as the iForma®, iUni® and iDuo® , offer advantages over the traditional "several-size-fits-all" approach such as a better fit, more natural movement, reduction in the amount of bone removed during surgery and a less invasive procedure. Such patient-specific implants generally can be created from images of the patient's joint. Based on the images, the patient-specific implant can be created both to include surfaces that match existing surfaces in the joint as well as to include surfaces that approximate an ideal and/or healthy surface that does not exist in the patient prior to any procedure. Implants also can be selected using imaging tests thereby optimizing the articular fit to select articular features or parameters.

### SUMMARY

The invention can be defined by the appending claims. Accordingly, the invention provides for a patient-specific femoral knee implant according to claims 1-15.

Certain embodiments provide method and devices to create a desired model of a joint or of portions or surfaces of a joint based on data derived from the existing joint. Data from the existing joint, for example, data generated from an image of the joint such as x-ray imaging, cone beam CT, digital tomosynthesis, and ultrasound, a MRI or CT scan or a PET or SPECT scan, is processed to generate a varied or corrected version of the joint or of portions of the joint or of surfaces within the joint. For example, the data can also be used to create a model that can be used to analyze the patient's joint and to devise and evaluate a course of corrective action. The data and/or model also can be used to design an implant component having one or more patient-specific aspects, such as a surface or curvature.

In one aspect, some embodiments provide implant components having an inner, bone-facing surface designed to negatively-match a bone surface that was cut, for example based on pre-determined geometries or based on patient-specific geometries. In certain embodiments, an outer joint-facing surface of a first implant component is designed to and/or does, at least in a portion of the component, optionally negatively, match an opposing outer joint-facing surface of a second implant component. By creating negatively-matching component surfaces at a joint interface, the opposing surfaces may not have an anatomic or near-anatomic shape, but instead may be negatively-matching or near-negatively matching to each other. This can have various advantages, such as reducing implant and joint wear and providing more predictable joint movement.

In another aspect, some embodiments provide implant components having one or more patient-specific curvatures or radii in one dimension, and one or more standard or engineered curvatures or radii in a second dimension.

In another aspect, some embodiments provide methods of designing, selecting, manufacturing, and implanting the patient-specific implant components.

It is to be understood that the features of the various embodiments described herein are not mutually exclusive and may exist in various combinations and permutations.

### BRIEF DESCRIPTION OF THE DRAWINGS

The foregoing and other objects, aspects, features, and advantages of embodiments will become more apparent and may be better understood by referring to the following description, taken in conjunction with the accompanying drawings, in which:
FIGS. A1 and A2 show schematic representations of a coronal plane cross section of a patient's distal femur (FIG. A1) and a femoral implant component (FIG. A2);
FIGS A3-A4 each show flow charts illustrating the process of designing, selecting and/or generating and installing patient specific or patient-engineered implants;

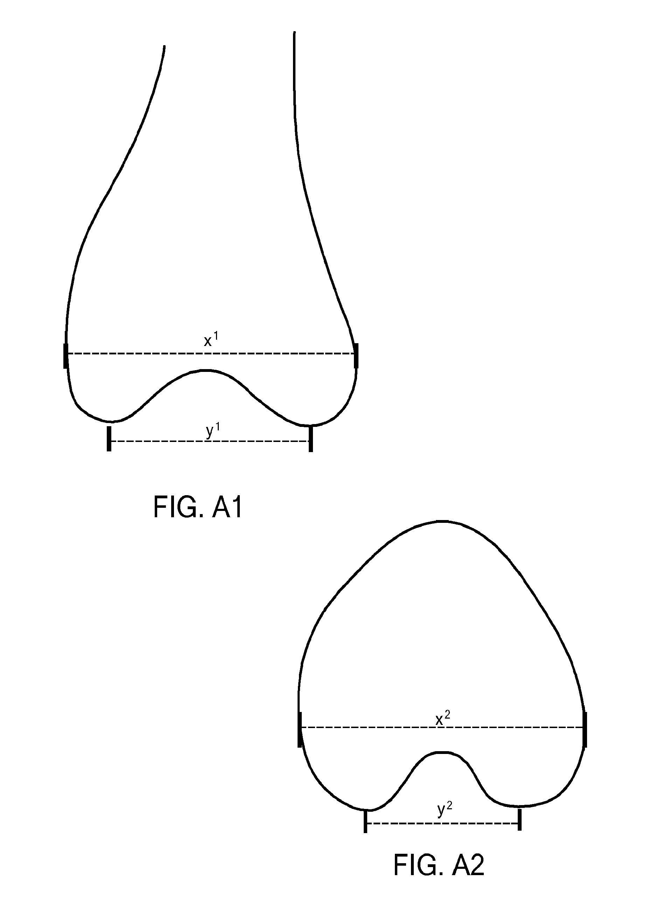

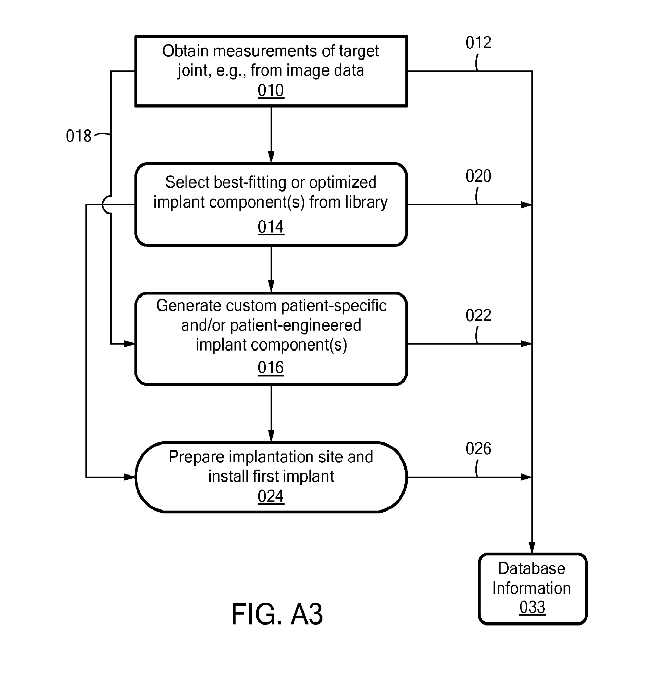

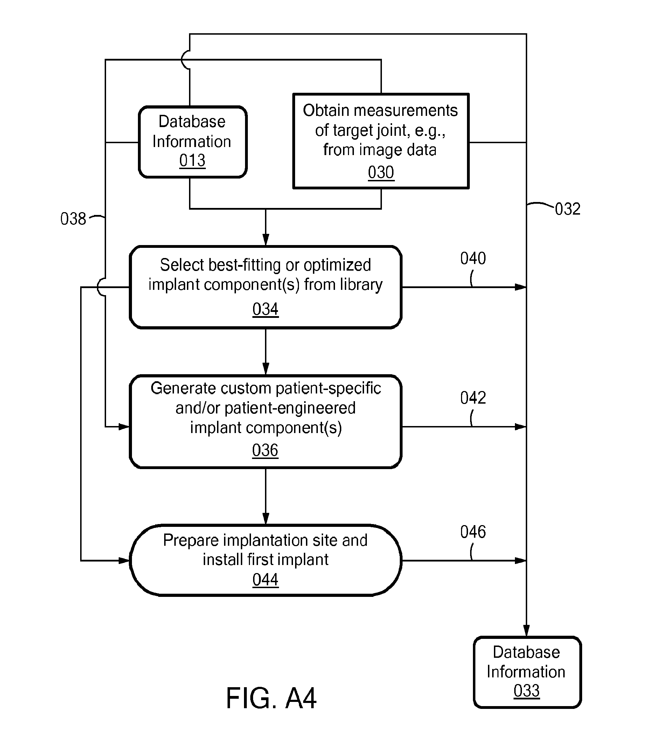

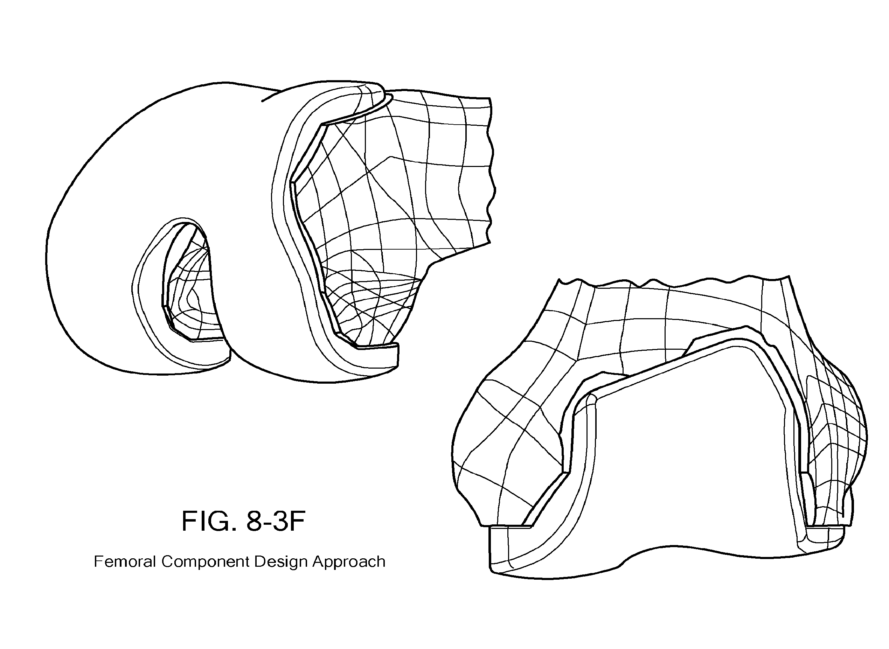

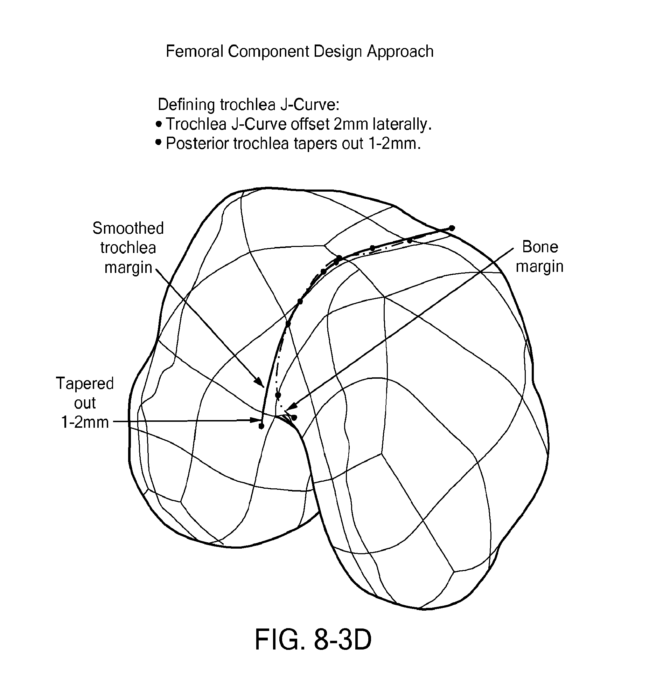

FIG. 8-3F shows an aspect of femoral component design approach;

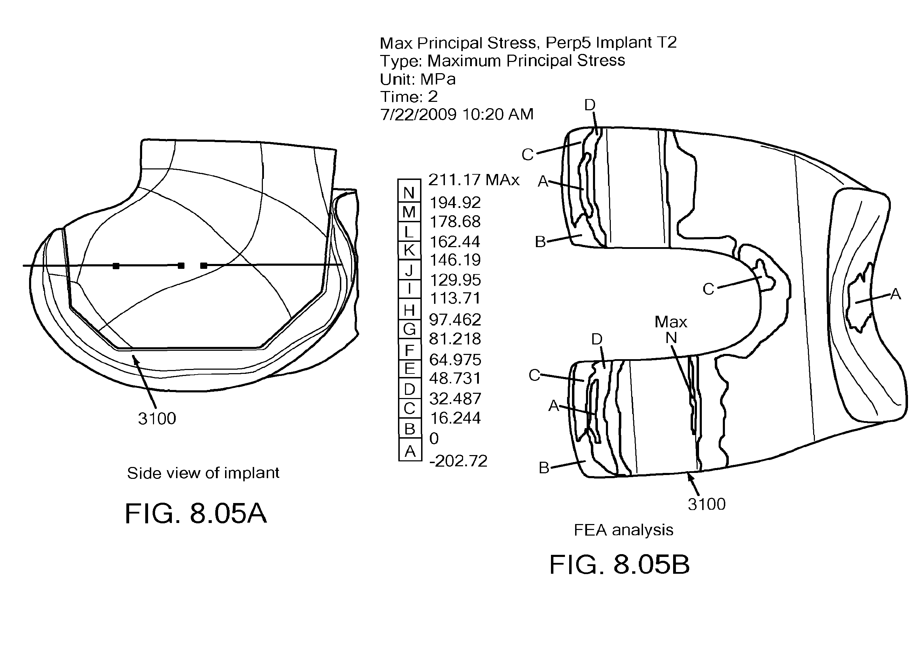

FIGS. 8.05A and 8.05B show a side view of an implant and the associated FEA analysis;

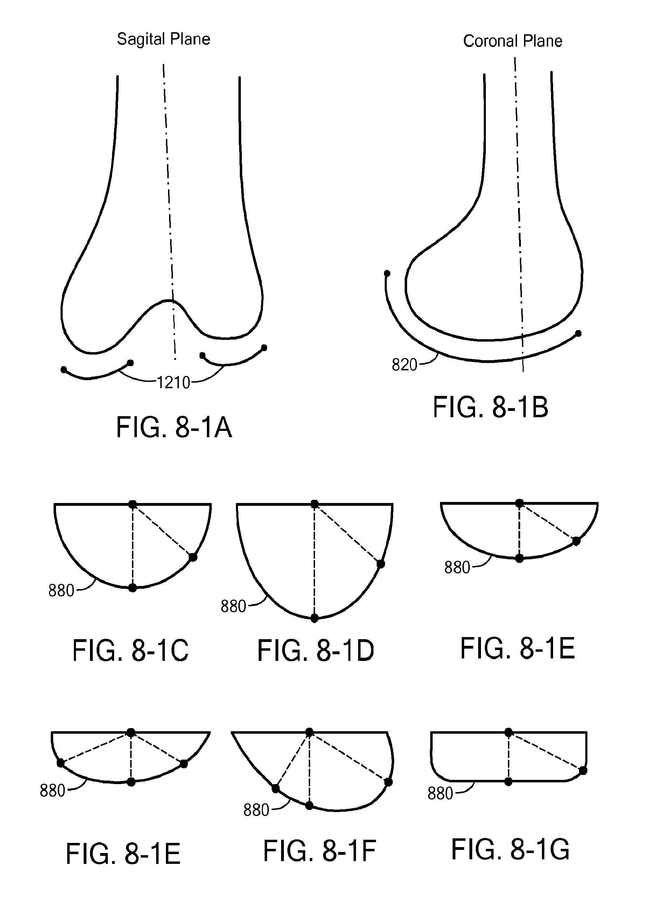

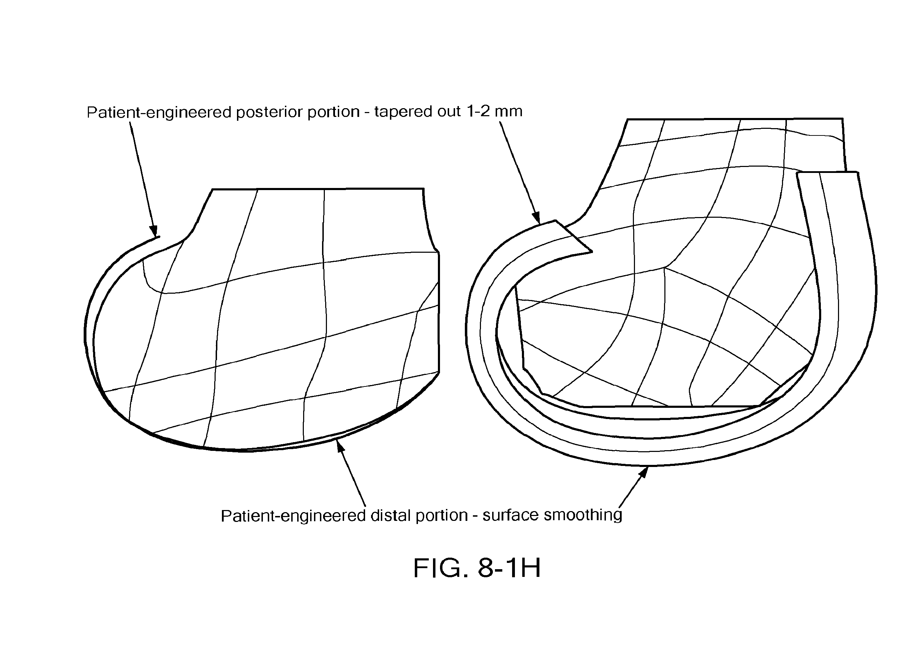

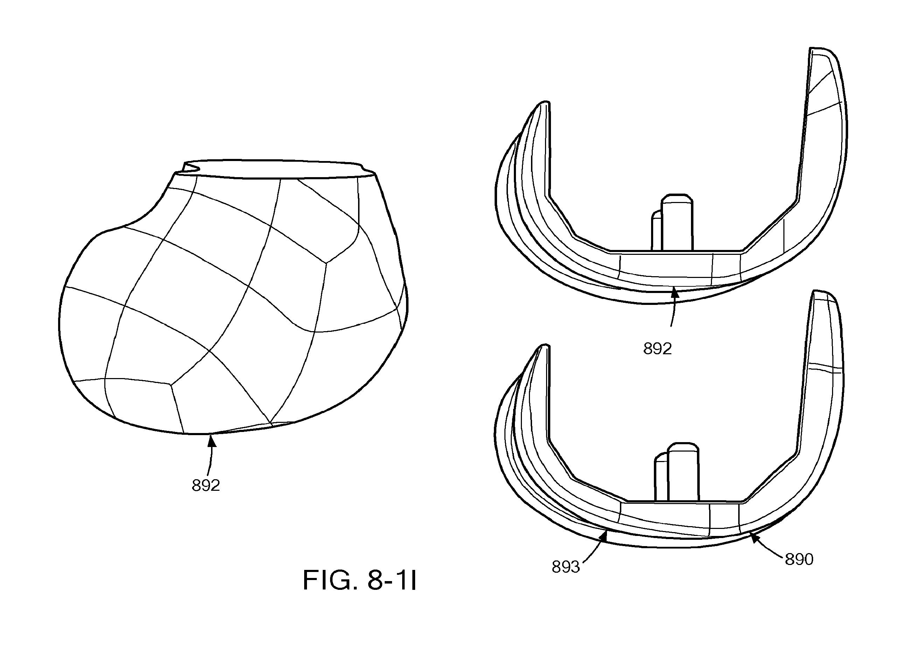

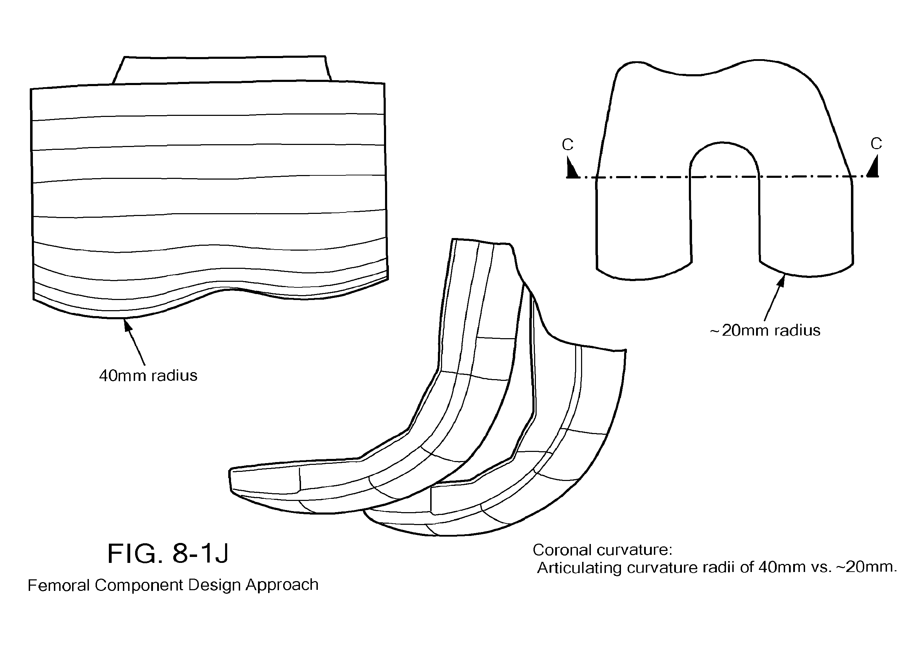

FIGS. 8-1A and 8-1B show the load bearing surfaces of a femoral implant component in a coronal view (FIG. 8-1A) and in a sagittal view (FIG. 8-1B);
FIGS. 8-1C-8-1J show tangent radii of various non-circular geometric shapes;
FIGS. 8-2A and 8-2B show cross-sections from a coronal view of two femoral condyle sections of a femoral component; FIG. 8-2C shows thicker material on a tibial implant component that is associated with designing the sagittal or j-curve of the femoral component to be tilted;
FIG. 8-3D shows a femoral component design approach;
FIG. 9-1A - 9-1C show three different bone cut for a femoral component;
FIGS. 9C1a through 9C3b show exemplary cross-sections of femoral implant components with bone cuts;
FIGS. 10-1A- 10-2P show implants designed for cruciate substitution;
FIGS. S6-1A - S6-13 show various guide tool configurations.

### DETAILED DESCRIPTION

### 1. Introduction

When a surgeon uses a traditional off-the-shelf implant to replace a patient's joint, for example, a knee joint, hip joint, or shoulder joint, certain spatial aspects of the implant typically do not match certain spatial aspects of the particular patient's biological structures at the joint.

Complications associated with mismatches can include pain, discomfort, soft tissue impingement, and an unnatural feeling of the joint during motion, e.g., so-called mid-flexion instability, as well as an altered range of movement and an increased likelihood of implant failure. Moreover, the loss of substantial portions of bone associated with implantation of a traditional primary implant typically limits the patient to only one subsequent revision implant.

The implants and methods of certain embodiments can be applied to any joint including, without limitation, a spine, spinal articulations, an intervertebral disk, a facet joint, a shoulder joint, an elbow, a wrist, a hand, a finger joint, a hip, a knee, an ankle, a foot, or a toe joint. Furthermore, various embodiments can be adapted and applied to implant instrumentation used during surgical or other procedures, and to methods of using various patient-specific implants, instruments, and other devices.

### 1.1 Patient-specific and patient-engineered

Certain embodiments relate to patient-specific implants and methods for designing, making, and using the same. Some embodiments relate to articular implant components having one or more patient-specific aspects adapted to a feature of the patient's biology, including one or more the patient's biological/anatomical structure, alignment, kinematics, and/or soft tissue impingements. The one or more patient-specific features of an implant component can include, but are not limited to, one or more implant component surfaces, such as surface contours, angles or bone cuts, and one or more implant component dimensions, such as thickness, width, depth, or length in one or more dimensions. The patient-specific aspect(s) of an implant component can be designed and manufactured (e.g., preoperatively designed and manufactured) based on patient-specific data to substantially match one or more of the patient's biological features. Alternatively or in addition, patient-specific aspect(s) of an implant component also can be achieved by analyzing imaging test data and selecting an implant that best fits one or more pre-determined patient specific parameters that are derived from the imaging test.

In addition, certain embodiments relate to patient-engineered implants and methods for designing, making, and using the same. Some embodiments relate to articular implant components having one or more patient-engineered aspects optimized from patient-specific data to meet one or more parameters to enhance a feature of the patient's biology, including one or more the patient's biological/anatomical structure, alignment, kinematics, and/or soft tissue impingements. As with the patient-specific aspect(s) of an implant component, the patient-engineered aspects of an implant component can be designed and manufactured (e.g., preoperatively designed and manufactured) or they can be selected, for example, by selecting an implant component that best meets the one or more predetermined parameters that enhance the feature of the patient's biology.

Moreover, an implant component can include a single patient-specific or patient-engineered feature that is both selected and designed (e.g., preoperatively selected and designed). For example, an implant component initially can be selected (e.g., from a library of implants) to have a feature with a larger dimension than the predetermined patient-specific or patient-engineered dimension. Then, the implant component can be machined (if selected from an actual library of implant components) or manufactured (if selected from a virtual library of implant components) so that the larger-dimensioned implant feature is altered to have the patient-specific or patient-engineered dimension.

### 1.2 Combinations of PS, PE, and standard features

A single implant component can include one or more patient-specific features, one or more patient-engineered features, and/or one or more standard (e.g., off-the-shelf features). The standard, off-the-shelf features can be selected to best fit with one or more of the patient-specific and/or patient-engineered features. For example, in a knee joint, a metal backed tibial component can be chosen that includes a standard locking mechanism and a patient adapted (i.e., patient-specific or patient-engineered) perimeter of the tibial component. A patient-specific perimeter of the tibial component can be achieved, for example, using cutting to match the patient's cortical bone perimeter in one or more dimensions of one more sections. Similarly, a polyethylene insert can be chosen that includes a standard locking mechanism, while the perimeter is adapted for better support to the patient's tibial bone perimeter or the perimeter of the metal backing.

In certain aspects, the implants and methods can include one or more patient-specific features and one or more standard features. For example, a curved surface of an implant component can include one or more dimensions or radii that are patient-specific, and one or more dimensions or radii that are standard. For example, in certain embodiments, a condyle portion of a femoral implant component and/or a corresponding groove in the bearing surface of a tibial implant component can include a patient-specific sagittal curvature or radii and a standard coronal curvature or radii. Coronal radii can also be patient derived, but an average or an optimum can be derived across the articular surface that is constant along the extent of the articular surface in the coronal plane. The patient-specific curvature or radii can be designed from patient-specific data to adapt to an existing feature of the patient's biology or it can be patient-engineered from patient-specific data to improve an existing feature of the patient's biology (e.g., a feature of the patient's biological structure, a distance or space between two biological structures, and/or a feature associated with the patient's anatomical function). Standard curvature or radii include curvatures or radii used in implants for all, or a collection of, patients.

In certain aspects, the implants and methods include a patient-specific inner surface for attaching to a patient's resected bone. In particular, patient-specific data collected preoperatively is used to determine one or more patient-specific bone cuts to a patient's bone and to the inner, bone-facing surface of an implant component. The bone cuts are optimized (i.e., patient-engineered) to maximize one or more parameters, such as: (1) deformity correction and limb alignment (2) maximizing preservation of bone, cartilage, or ligaments, (3) maximizing preservation and/or optimization of other features of the patient's anatomy, such as trochlea and trochlear shape, (4) restoration and/or optimization of joint kinematics, and/or (5) restoration or optimization of joint-line location and/or joint gap width. Based on the optimized bone cuts and, optionally, outer implant shape parameters and features, the implant's inner, bone-facing surface is designed to, at least in part, negatively-match the shape of the cut bone. In addition, the implant's outer, joint-facing surface can be designed to, at least in part, substantially negatively-match the opposing surface at the joint cavity. The outer surface can include patient-specific features, e.g., a patient-specific sagittal curvature and a patient-engineered coronal curvature, or a patient-specific sagittal curvature and a patient-specific coronal curvature. These curvatures can be patient-specific in at least a portion or the entire outer surface of the implant. These curvatures can be patient-specific in one condyle, but not patient specific in the other condyle. The shape of a first condyle can be, for example, used to generate a shape of a second condyle.

### 1.3 Implant system

An implant (i.e., an implant system) can be designed and/or selected to include one or more patient specific implant components, one or more patient-engineered implant components, and one or more standard (e.g., off-the-shelf) implant components. For example, a femoral implant component can be patient-specific and can be combined with an off-the-shelf tibial implant component. In this example, the entire tibial implant component can be off-the-shelf. Alternatively, a metal backed implant component can be patient specific, e.g., matched in the A-P dimension or the M-L dimension to the patient's tibial cortical bone, while a plastic insert can include a standard off-the-shelf configuration. Off-the-shelf configuration can mean that the tibial insert has fixed, standard dimensions to fit, for example, into a standard tibial tray. Off-the-shelf configuration also can mean that the tibial insert has a fixed, standard dimension or distance between two tibial dishes or curvatures to accommodate the femoral bearing surface. The latter configuration is particularly applicable in an implant system that uses a femoral implant component that is patient-specifically matched in the M-L dimension to the distal femur of the patient's bone, but uses a standardized intercondylar notch width on the femoral component to achieve optimal mating with a corresponding tibial insert. For example, FIGS. A1 and A2 show schematic representations of a coronal plane cross section of a patient's distal femur (FIG. A1) and a femoral implant component (FIG. A2). As shown in the figure, the M-L dimension, x^2, of the implant component matches the M-L dimension, x^1, of the patient's femur. However, the intercondylar notch width, y^2, of the implant component is smaller than the patient's intercondylar notch width, y^1. Specifically, intercondylar notch width, y^2, of the implant component is designed to be a standard distance, for example, so that is can properly engage during joint motion a tibial insert having a standard engaging surface.

### 1.4 Introduction of pre-primary

Certain embodiments are directed to patient-specific implants and implant designs applied as a pre-primary implant device, such that a subsequent, replacement implant can be performed with a second (and, optionally, a third) patient-specific pre-primary implant device or with a traditional primary implant. Certain embodiments are directed to patient-specific implants and implant designs applied as a primary implant device, such that a subsequent, replacement implant can be performed as a traditional revision. Certain embodiments are directed to patient-specific implants and implant designs applied as a revision implant device, such that a subsequent revision may be possible with a second, patient-specific implant having one or more patient-specific aspects.

In one embodiment, a first patient specific and/or patient-engineered implant can be designed or selected. The patient-specific implant includes at least one or more features in its dimensions or shape or outer surface that are patient-specific. The shape information of the implant can be stored in a database. When the implant fails, e.g., due to bone loss or osteolysis or aseptic loosening at a later point in time, e.g., 15 years after the original implantation, a second implant can be implanted.

FIG. A3 is a flow chart illustrating the process of designing, selecting and/or generating, and installing a first patient-specific or patient-engineered implant. First, using the techniques described herein or those suitable and known in the art, measurements of the target joint are obtained 010. This step can be repeated multiple times, as desired. Optionally, a virtual model of the joint can be generated, for example, to determine the proper alignment of the joint and the corresponding resection cuts and implant component features based on the proper alignment of the joint. This information can be collected and stored 012 in a database 013. Once measurements of the target joint are obtained and analyzed to determine resection cuts and patient-specific or optimized implant features, the implant components can be selected 014 and or generated 016 (e.g., virtual selection and/or generation and then physical selection and/or generation). Specifically, one or more implant components with best-fitting and/or optimized features can be selected 014 and then further customized 016. Alternatively or in addition, one or more implant components with best-fitting and/or optimized features can be customized without an initial selection from a library 018. Using a virtual model to assess the selected or customized implant component, this process also can be repeated as desired (e.g., before one or more physical components are selected and/or generated). The information regarding the selected and/or generated implant components can be collected and stored 020, 022 in a database 013. Once a desired implant component or set of implant components is obtained, a surgeon can prepare the implantation site and install the first implant 024. The information regarding preparation of the implantation site and implant installation can be collected and stored 026 in a database 013.

FIG. A4 is a flow chart illustrating the process of designing, selecting and/or generating, and installing a second implant. The steps are similar to those described above for a first implant; however, in the second implant process, the database information 013 collected and stored in the first implant process can be used as part of the process for the second implant. First, in addition to the database information from the first implant process, additional measurements of the target joint with the first implant optionally can be obtained 030 and used together with the database information 013 from the first implant process. This step can be repeated multiple times, as desired. Optionally, a virtual model of the joint and first implant can be generated, for example, to determine proper joint alignment and corresponding resection cuts and second implant component features based on the proper alignment. This information can be collected and stored 032 as new or additional database information 033. Once the database information from the first implant process, and optionally new measurements of the target joint and first implant, are obtained and analyzed to determine resection cuts and patient-specific or optimized implant features, the implant components for the second implant can be selected 034 and or generated 036 (e.g., virtual selection and/or generation and then physical selection and/or generation). Specifically, one or more implant components with best-fitting and/or optimized features for the second implant can be selected 034 and then further customized 036. Alternatively or in addition, one or more implant components with best-fitting and/or optimized features for the second implant can be customized without an initial selection from a library 038. Using a virtual model to assess the selected or customized implant component, this process also can be repeated as desired (e.g., before one or more physical components are selected and/or generated). The information regarding the selected and/or generated implant components for the second implant can be collected and stored 040, 042 in a database 033. Once a desired implant component or set of implant components is obtained for the second implant, a surgeon can prepare the implantation site, including removing the first implant, and install the second implant 044. The information regarding preparation of the implantation site and second implant installation can be collected and stored 046 in a database 033.

The second implant can have standard attachment mechanisms, e.g., a stem and or pegs or other attachment means known in the art. Alternatively, the attachment mechanisms can be patient-specific by deriving shape information on the residual bone, e.g., of a femur and acetabulum or of a femur and a tibia or of a humerus and a glenoid, using image data, e.g., CT or MRI data. The outer surface or outer shape of the second implant can be adapted to include, at least in part, information reflective of the dimensions or shape or outer surface of the first implant. In this manner, a better functional result can be achieved with the revision implant by maintaining patient specific shapes and/or geometry in the revision implant by accessing data in the patient database.

Accordingly, certain embodiments described herein are directed to implant, implant components, and methods that address many of the problems associated with traditional implants, such as mismatches between an implant component and a patient's biological features (e.g., a feature of a biological structure, a distance or space between two biological structures, and/or a feature associated with anatomical function) and substantial bone removal that limits subsequent revisions following a traditional primary implant.

### 2. Exemplary implant systems and patient-specific features

The concepts described herein can be embodied in various types of implants and implant systems including, but not limited to, knee-joint implants, hip-joint implants, and shoulder-joint implants. In certain embodiments, an implant or implant system can include one, two, three, four or more components. An implant component can be designed and/or manufactured to include one or more patient-specific features that substantially match one or more of the patient's anatomical/biological structures, such as bone, cartilage, tendon, or muscle. In addition or alternatively, an implant component can be designed and/or manufactured to include one or more patient-specific features that substantially match one or more other implant components. In addition, an implant component can be designed and/or manufactured to include one or more non-patient-specific features that substantially match one or more other implant components. Exemplary combinations are provided in **Table 1.**

**Table 1: Exemplary combinations of patient specific (P), patient-engineered (PE), and standard (St) features¹ in implant systems**

| | **Implant component feature number²** | | | | | | | | | | | | |
|---|---|---|---|---|---|---|---|---|---|---|---|---|---|
| **Implant system number** | 1 | 2 | 3 | 4 | 5 | 6 | 7 | 8 | 9 | 10 | 11 | 12 | 13 |
| **1** | P | P | P | P | P | P | P | P | P | P | P | P | P |
| **2** | PE | PE | PE | PE | PE | PE | PE | PE | PE | PE | PE | PE | PE |
| **3** | St | St | St | St | St | St | St | St | St | St | St | St | St |
| **4** | P | St | St | St | St | St | St | St | St | St | St | St | St |
| **5** | P | P | St | St | St | St | St | St | St | St | St | St | St |
| **6** | P | P | P | St | St | St | St | St | St | St | St | St | St |
| **7** | P | P | P | P | St | St | St | St | St | St | St | St | St |
| **8** | P | P | P | P | P | St | St | St | St | St | St | St | St |
| **9** | P | P | P | P | P | P | St | St | St | St | St | St | St |
| **10** | P | P | P | P | P | P | P | St | St | St | St | St | St |
| **11** | P | P | P | P | P | P | P | P | St | St | St | St | St |
| **12** | P | P | P | P | P | P | P | P | P | St | St | St | St |
| **13** | P | P | P | P | P | P | P | P | P | P | St | St | St |
| **14** | P | P | P | P | P | P | P | P | P | P | P | St | St |
| **15** | P | P | P | P | P | P | P | P | P | P | P | P | St |
| **16** | P | PE | PE | PE | PE | PE | PE | PE | PE | PE | PE | PE | PE |
| **17** | P | P | PE | PE | PE | PE | PE | PE | PE | PE | PE | PE | PE |
| **18** | P | P | P | PE | PE | PE | PE | PE | PE | PE | PE | PE | PE |
| **19** | P | P | P | P | PE | PE | PE | PE | PE | PE | PE | PE | PE |
| **20** | P | P | P | P | P | PE | PE | PE | PE | PE | PE | PE | PE |
| **21** | P | P | P | P | P | P | PE | PE | PE | PE | PE | PE | PE |
| **22** | P | P | P | P | P | P | P | PE | PE | PE | PE | PE | PE |
| **23** | P | P | P | P | P | P | P | P | PE | PE | PE | PE | PE |
| **24** | P | P | P | P | P | P | P | P | P | PE | PE | PE | PE |
| **25** | P | P | P | P | P | P | P | P | P | P | PE | PE | PE |
| **26** | P | P | P | P | P | P | P | P | P | P | P | PE | PE |
| **27** | P | P | P | P | P | P | P | P | P | P | P | P | PE |
| **28** | PE | St | St | St | St | St | St | St | St | St | St | St | St |
| **29** | PE | PE | St | St | St | St | St | St | St | St | St | St | St |
| **30** | PE | PE | PE | St | St | St | St | St | St | St | St | St | St |
| **31** | PE | PE | PE | PE | St | St | St | St | St | St | St | St | St |
| **32** | PE | PE | PE | PE | PE | St | St | St | St | St | St | St | St |
| **33** | PE | PE | PE | PE | PE | PE | St | St | St | St | St | St | St |
| **34** | PE | PE | PE | PE | PE | PE | PE | St | St | St | St | St | St |
| **35** | PE | PE | PE | PE | PE | PE | PE | PE | St | St | St | St | St |
| **36** | PE | PE | PE | PE | PE | PE | PE | PE | PE | St | St | St | St |
| **37** | PE | PE | PE | PE | PE | PE | PE | PE | PE | PE | St | St | St |
| **38** | PE | PE | PE | PE | PE | PE | PE | PE | PE | PE | PE | St | St |
| **39** | PE | PE | PE | PE | PE | PE | PE | PE | PE | PE | PE | PE | St |
| **40** | P | PE | St | St | St | St | St | St | St | St | St | St | St |
| **41** | P | PE | PE | St | St | St | St | St | St | St | St | St | St |
| **42** | P | PE | PE | PE | St | St | St | St | St | St | St | St | St |
| **43** | P | PE | PE | PE | PE | St | St | St | St | St | St | St | St |
| **44** | P | PE | PE | PE | PE | PE | St | St | St | St | St | St | St |
| **45** | P | PE | PE | PE | PE | PE | PE | St | St | St | St | St | St |
| **46** | P | PE | PE | PE | PE | PE | PE | PE | St | St | St | St | St |
| **47** | P | PE | PE | PE | PE | PE | PE | PE | PE | St | St | St | St |
| **48** | P | PE | PE | PE | PE | PE | PE | PE | PE | PE | St | St | St |
| **49** | P | PE | PE | PE | PE | PE | PE | PE | PE | PE | PE | St | St |
| **50** | P | PE | PE | PE | PE | PE | PE | PE | PE | PE | PE | PE | St |
| **51** | P | P | PE | St | St | St | St | St | St | St | St | St | St |
| **52** | P | P | PE | PE | St | St | St | St | St | St | St | St | St |
| **53** | P | P | PE | PE | PE | St | St | St | St | St | St | St | St |
| **54** | P | P | PE | PE | PE | PE | St | St | St | St | St | St | St |
| **55** | P | P | PE | PE | PE | PE | PE | St | St | St | St | St | St |
| **56** | P | P | PE | PE | PE | PE | PE | PE | St | St | St | St | St |
| **57** | P | P | PE | PE | PE | PE | PE | PE | PE | St | St | St | St |
| **58** | P | P | PE | PE | PE | PE | PE | PE | PE | PE | St | St | St |
| **59** | P | P | PE | PE | PE | PE | PE | PE | PE | PE | PE | St | St |
| **60** | P | P | PE | PE | PE | PE | PE | PE | PE | PE | PE | PE | St |
| **61** | P | P | P | PE | St | St | St | St | St | St | St | St | St |
| **62** | P | P | P | PE | PE | St | St | St | St | St | St | St | St |
| **63** | P | P | P | PE | PE | PE | St | St | St | St | St | St | St |
| **64** | P | P | P | PE | PE | PE | PE | St | St | St | St | St | St |
| **65** | P | P | P | PE | PE | PE | PE | PE | St | St | St | St | St |
| **66** | P | P | P | PE | PE | PE | PE | PE | PE | St | St | St | St |
| **67** | P | P | P | PE | PE | PE | PE | PE | PE | PE | St | St | St |
| **68** | P | P | P | PE | PE | PE | PE | PE | PE | PE | PE | St | St |
| **69** | P | P | P | PE | PE | PE | PE | PE | PE | PE | PE | PE | St |
| **70** | P | P | P | P | PE | St | St | St | St | St | St | St | St |
| **71** | P | P | P | P | PE | PE | St | St | St | St | St | St | St |
| **72** | P | P | P | P | PE | PE | PE | St | St | St | St | St | St |
| **73** | P | P | P | P | PE | PE | PE | PE | St | St | St | St | St |
| **74** | P | P | P | P | PE | PE | PE | PE | PE | St | St | St | St |
| **75** | P | P | P | P | PE | PE | PE | PE | PE | PE | St | St | St |
| **76** | P | P | P | P | PE | PE | PE | PE | PE | PE | PE | St | St |
| **77** | P | P | P | P | PE | PE | PE | PE | PE | PE | PE | PE | St |
| **78** | P | P | P | P | P | PE | St | St | St | St | St | St | St |
| **79** | P | P | P | P | P | PE | PE | St | St | St | St | St | St |
| **80** | P | P | P | P | P | PE | PE | PE | St | St | St | St | St |
| **81** | P | P | P | P | P | PE | PE | PE | PE | St | St | St | St |
| **82** | P | P | P | P | P | PE | PE | PE | PE | PE | St | St | St |
| **83** | P | P | P | P | P | PE | PE | PE | PE | PE | PE | St | St |
| **84** | P | P | P | P | P | PE | PE | PE | PE | PE | PE | PE | St |
| **85** | P | P | P | P | P | P | PE | St | St | St | St | St | St |
| **86** | P | P | P | P | P | P | PE | PE | St | St | St | St | St |
| **87** | P | P | P | P | P | P | PE | PE | PE | St | St | St | St |
| **88** | P | P | P | P | P | P | PE | PE | PE | PE | St | St | St |
| **89** | P | P | P | P | P | P | PE | PE | PE | PE | PE | St | St |
| **90** | P | P | P | P | P | P | PE | PE | PE | PE | PE | PE | St |
| **91** | P | P | P | P | P | P | P | PE | St | St | St | St | St |
| **92** | P | P | P | P | P | P | P | PE | PE | St | St | St | St |
| **93** | P | P | P | P | P | P | P | PE | PE | PE | St | St | St |
| **94** | P | P | P | P | P | P | P | PE | PE | PE | PE | St | St |
| **95** | P | P | P | P | P | P | P | PE | PE | PE | PE | PE | St |
| **96** | P | P | P | P | P | P | P | P | PE | St | St | St | St |
| **97** | P | P | P | P | P | P | P | P | PE | PE | St | St | St |
| **98** | P | P | P | P | P | P | P | P | PE | PE | PE | st | St |
| **99** | P | P | P | P | P | P | P | P | PE | PE | PE | PE | st |
| **100** | P | P | P | P | P | P | P | P | P | PE | St | St | St |
| **101** | P | P | P | P | P | P | P | P | P | PE | PE | St | St |
| **102** | P | P | P | P | P | P | P | P | P | PE | PE | PE | St |
| **103** | P | P | P | P | P | P | P | P | P | P | PE | St | St |
| **104** | P | P | P | P | P | P | P | P | P | P | PE | PE | St |
| **105** | P | P | P | P | P | P | P | P | P | P | P | PE | St |

| | | | | | | | | | | | | | |
|---|---|---|---|---|---|---|---|---|---|---|---|---|---|
| 1: S = standard, off the shelf, P = patient specific, PE = patient engineered (e.g., constant coronal curvature, derived off patient's coronal curvatures along articular surface) 2: Each of the thirteen numbered implant features represents a different exemplary implant feature, for example, one of (1) femoral implant component M-L dimension, (2) femoral implant component A-P dimension, (3) femoral implant component bone cut, (4) femoral implant component sagittal curvature, (5) femoral implant component coronal curvature, (6) femoral implant component inter-condylar distance, (7) femoral implant component notch location / geometry, (8) tibial implant component M-L dimension, (9) tibial implant component A-P dimension, (10) tibial implant component insert inter-condylar distance, (11) tibial implant component insert lock, (12) tibial implant component metal backing lock, and (13) tibial implant component metal backing perimeter. | | | | | | | | | | | | | |

### 2.1 Libraries

In certain embodiments, a library can be generated to include images from a particular patient at one or more ages prior to the time that the patient needs a joint implant. For example, a method can include identifying patients eliciting one or more risk factors for a joint problem, such as low bone mineral density score, and collecting one or more images of the patient's joints into a library. In certain embodiments, all patients below a certain age, for example, all patients below 40 years of age can be scanned to collect one or more images of the patient's joint. The images and data collected from the patient can be banked or stored in a patient-specific database. For example , the articular shape of the patient's joint or joints can be stored in an electronic database until the time when the patient needs an implant. Then, the images and data in the patient specific database can be accessed and a patient specific and/or patient-engineered partial or total joint replacement implant using the patient's originally anatomy, not affected by arthritic deformity yet, can be generated. This process results is a more functional and more anatomic implant.

### 2.2 Selecting an implant from a library

In certain embodiments, an implant component can be selected from a library of implant components in a manner to achieve a patient-specific fit and/or an optimal fit with the implantation site. The fit can be optimized with regard to any number of patient-specific measurements, including one or more of the exemplary measurements described in **Table 2.**

**Table 2: Exemplary patient-specific measurements of anatomical features that can be used in the selection and/or design of an implant**

| **Anatomical feature** | **Exemplary measurement** |
|---|---|
| **Joint line, joint gap** | - Location relative to proximal reference point |
| | - Location relative to distal reference point |
| | - Angle |
| | - Gap distance between opposing surfaces in one or more locations |
| | - Location, angle, and/or distance relative to contralateral joint |
| **Soft tissue tension and/or balance** | - Joint gap distance |
| | - Joint gap differential, e.g., medial to lateral |
| **Medullary cavity** | - Shape in one or more dimensions |
| | - Shape in one or more locations |
| | - Diameter of cavity |
| | - Volume of cavity |
| **Subchondral bone** | - Shape in one or more dimensions |
| | - Shape in one or more locations |
| | - Thickness in one or more dimensions |
| | - Thickness in one or more locations |
| | - Angle, e.g., resection cut angle |
| **Cortical bone** | - Shape in one or more dimensions |
| | - Shape in one or more locations |
| | - Thickness in one or more dimensions |
| | - Thickness in one or more locations |
| | - Angle, e.g., resection cut angle |
| **Endosteal bone** | - Shape in one or more dimensions |
| | - Shape in one or more locations |
| | - Thickness in one or more dimensions |
| | - Thickness in one or more locations |
| | - Angle, e.g., resection cut angle |
| **Cartilage** | - Shape in one or more dimensions |
| | - Shape in one or more locations |
| | - Thickness in one or more dimensions |
| | - Thickness in one or more locations |
| | - Angle, e.g., resection cut angle |
| **Intercondylar notch** | - Shape in one or more dimensions |
| | - Location |
| | - Height in one or more locations |
| | - Width in one or more locations |
| | - Depth in one or more locations |
| | - Angle, e.g., resection cut angle |
| **Medial condyle** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Lateral condyle** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Trochlea** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Medial trochlea** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Central trochlea** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Lateral trochlea** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Entire tibia** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Medial tibia** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Lateral tibia** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Entire patella** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Medial patella** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Central patella** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Lateral patella** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Femoral head** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Acetabulum** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Glenoid** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Humeral head** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Ankle joint** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Elbow** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Wrist** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Hand** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Finger** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle |
| **Spine** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Facet joint** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |

The patient-specific measurements selected for the evaluation can be used to match or optimize from the library the selected implant features, including one or more of the exemplary features described in **Table 3.** The library can be a virtual library of implant components or a physical library of implant components.

**Table 3: Exemplary implant features that can be selected and/or designed based on patient-specific measurements**

| **Category** | **Exemplary feature** |
|---|---|
| **Implant or implant or component (applies to most implants and implant components)** | - One or more portions of, or all of, an external implant component curvature |
| | - One or more portions of, or all of, an internal implant dimension |
| | - One or more portions of, or all of, an internal or external implant angle |
| | - Portions or all of one or more of the ML, AP, SI dimension of the internal and external component and component features |
| | - An outer locking mechanism dimension between a plastic or non-metallic insert and a metal backing component in one or more dimensions |
| | - Component height |
| | - Component profile |
| | - Component 2D or 3D shape |
| | - Component volume |
| | - Composite implant height |
| | - Insert width |
| | - Insert shape |
| | - Insert length |
| | - Insert height |
| | - Insert profile |
| | - Insert curvature |
| | - Insert angle |
| | - Distance between two curvatures or concavities |
| | - Polyethylene or plastic width |
| | - Polyethylene or plastic shape |
| | - Polyethylene or plastic length |
| | - Polyethylene or plastic height |
| | - Polyethylene or plastic profile |
| | - Polyethylene or plastic curvature |
| | - Polyethylene or plastic angle |
| | - Component stem width |
| | - Component stem shape |
| | - Component stem length |
| | - Component stem height |
| | - Component stem profile |
| | - Component stem curvature |
| | - Component stem position |
| | - Component stem thickness |
| | - Component stem angle |
| | - Component peg width |
| | - Component peg shape |
| | - Component peg length |
| | - Component peg height |
| | - Component peg profile |
| | - Component peg curvature |
| | - Component peg position |
| | - Component peg thickness |
| | - Component peg angle |
| | - Slope of an implant surface |
| | - Number of sections, facets, or cuts on an implant surface |
| **Femoral implant or implant component** | - Condylar distance of a femoral component, e.g., between femoral condyles |
| | - A condylar coronal radius of a femoral component |
| | - A condylar sagittal radius of a femoral component |
| **Tibial implant or implant component** | - Slope of an implant surface |
| | - Condylar distance, e.g., between tibial joint-facing surface concavities that engage femoral condyles |
| | - Coronal curvature (e.g., one or more radii of curvatures in the coronal plane) of one or both joint-facing surface concavities that engage each femoral condyle |
| | - Sagittal curvature (e.g., one or more radii of curvatures in the sagittal plane) of one or both joint-facing surface concavities that engage each femoral condyle |

In certain embodiments, a combination of parameters can be selected from the library. For example, one or more of an M-L measurement, an A-P measurement, and an S-I measurement o a patient's joint can be obtained from the subject preoperatively, for example, from one or more images of the subject's joint. Then, based on the one or measurements, an implant or implant component for the subject's joint can be designed or selected preoperatively.

### 2.3 Using threshold values

This process also can include generating and/or using a model, for example, a virtual model, of the patient's joint that includes the selected measurements and virtually fitting one or more designed or selected implants into the virtual model. This approach allows for iterative design and/or selection improvement and can include steps to virtually assess the fit, such as virtual kinematics assessment.

One or more thresholds and/or weightings can be applied for the selection and/or matching process. Different parameters can have the same weighting or they can have a different weighting. A parameter can include one or more thresholds for selecting one or more implants. The thresholds can include one or more minimum threshold values, for example, 80%, greater than 80%, 85%, greater than 85%, 90%, greater than 90%, 95%, greater than 95%, 97%, greater than 97%, 98%, greater than 98%, 99%, greater than 99%, 100%, and/or greater than 100% a target value, such as minimum implant coverage of a certain anatomical structure or feature. Alternatively or additionally, the thresholds can include one or more maximum threshold values, such as 105%, less than 105%, 103%, less than 103%, 102%, less than 102%, 101%, less than 101%, 100%, and/or less than 100% a target value, such as maximum implant coverage of a certain anatomical structure or feature.

One or more parameter thresholds can be absolute, for example, in a selection and/or matching process that selects only those implants that meet a minimum threshold for a particular patient of 95% mediolateral femoral condyle coverage all around the condyle(s). An example having multiple absolute thresholds is a selection and/or matching process that selects only those implants from a library that meet both a minimum threshold for a particular patient of 95% mediolateral femoral condyle coverage in the central weight-bearing region, and a minimum threshold of greater than 80% mediolateral femoral condyle coverage outside the weight-bearing area.

Alternatively or in addition, one or more parameter thresholds can be contingent on one or more other factors. In particular, a selection and/or matching process can successively search a library for implants based on contingent thresholds. For example, implants meeting a minimum threshold of 99% mediolateral femoral condyle coverage initially can be selected. If no implant meets the threshold, or if some implants meet the threshold but do not meet other parameter thresholds, then a second selection round can include implants meeting a minimum threshold of 98% mediolateral femoral condyle coverage. The process can continue to use additional, contingent thresholds until an implant with the selected parameter thresholds is identified.

Different thresholds can be defined in different anatomic regions and for different parameters. For example, the amount of mediolateral tibial implant coverage can be set at 90%, while the amount of anteroposterior tibial implant coverage can be set at 85%. In another example, the congruency in intercondylar notch shape can be set at 80% required, while the required mediolateral condylar coverage can be set at 95%.

### 2.4 Designing or selecting a femoral implant component

In certain embodiments, the size of an implant component can be designed and/or selected to substantially match the size of a patient's corresponding biological structure. For example, the width and/or height of one or more dimensions of a femoral implant component can be preoperatively designed and/or selected to substantially match the corresponding dimension(s) of a patient's distal femur as determined, for example, from one or more images of the patient's joint. In certain embodiments, the size of the femoral implant component can be designed and/or selected based on one or more patient-specific dimensions, such as the length of the patient's epicondylar axis, which can be determined from preoperatively collected patient data, such as image data.

In certain embodiments, the area of the implant component can be designed and/or selected preoperatively to be patient-specific, in whole or in part. For example, as shown in FIG. 8-3F, the surface area of all or part of the bone-facing surface of the implant component can be designed and/or selected from patient-specific data to substantially match the corresponding resected surface area of the patient's femur In certain embodiments, the implant component substantially covers the resected surface area, for example, by covering up to 100% and at least 90%, greater than 90%, at least 95%, greater than 95%, at least 97%, greater than 97%, at least 98%, greater than 98%, at least 99%, and/or greater than 99% of the resected surface area of the patient's distal femur.

In certain embodiments, the thickness of one or portions of an implant component can be designed and/or selected to match the corresponding dimension of the patient's biological structure. For example, the implant component thickness can be designed and/or selected to substantially match in one or more sections the thickness of the patient's cartilage, the thickness of the patient's resected bone, the total thickness of the patient's cartilage and resected bone, or an optimized thickness based on one or more of the parameters described above. In certain embodiments, the additive thickness of two or more implant components, for example, femoral and tibial implant components, can be designed and/or selected together to substantially match in one or more sections the patient's joint-gap distance, for example, the distance between the patient's femoral and tibial bone surfaces, uncut or resected.

In certain embodiments, the implant component design and/or selection can include a thickness, a minimum implant thickness and/or a maximum implant thickness that is engineered from patient-specific data to provide to the patient an optimized implant fit with respect to one or more parameters, for example, as described above. Moreover, the additive thickness of two or more implant components can be engineered from patient-specific data to optimize the implant thickness for meeting one or more of the parameters described above. For example, the femoral and/or tibial implant thicknesses can be engineered from patient-specific data to optimize or correct the patient's joint-gap distance. The patient-specific data that may be used to engineer a minimum implant thickness can include, for example, one or more of patient height, patient weight, patient age, patient activity level, patient joint-size (e.g., epicondylar distance or condylar width), and other patient-specific data.

In preferred embodiments, the minimum implant component thickness can be engineered based on, or together with, other patient-engineered dimensions or aspects of the implant component, for example, one or more of implant component size, implant component condyle width, and one or more implant component surface curvatures. For example, the thinnest part of a femoral implant component frequently appears at the intersection of the implant component's distal bone cut and posterior bone cut, as shown by the arrow in FIG. 8.05A. This portion of an implant component also frequently shows the highest stress load, as exemplified by the FEA analysis results shown in FIG. 8.05B. Accordingly, the minimum implant component thickness for this or any portion of the implant component can be engineered based on one or more factors related to stress load, for example: the size of the patient; the size of the patient's femur; the size of the patient's condyle; the size of the patient-engineered implant component; the size of the implant component condyle; and the implant component's joint-facing surface curvature in the region that is opposite the intersection of the distal bone cut and posterior bone cut. In this way, a patient-specific minimum implant thickness can be engineered from patient-specific data, such as image data, and designed into the patient-adapted implant. This allows for minimal bone to be resected in the implant procedure and thereby can help to maximize bone preservation for any particular patient. Preservation of bone can allow for a subsequent knee implant to be a primary knee implant procedure, rather than a revision procedure.

In addition or alternatively, the implant component can be designed to include a standard minimum thickness and/or a standard maximum thickness in one or more locations. For example, in certain embodiments, the implant component can include a minimum implant thickness of 9 mm, less than 9 mm, 8 mm, less than 8 mm, 7 mm, less than 7 mm, 6 mm, less than 6 mm, 5mm, and/or less than 5 mm.

In certain embodiments, the joint-facing surface of a femoral implant component includes one or more patient-specific dimensions that positively or negatively-match the patient's biological structure or that are engineered from patient-specific data to provide to the patient an optimized implant fit with respect to one or more parameters, for example, as described above. For example, a femoral implant component can be designed and/or selected to include a joint-facing surface that substantially positively-matches one or more dimensions of the patient's femoral joint-facing surface, for example, as determined by preoperatively collected data, such as image data. The patient's femoral joint-facing surface that is included in the image data can include, for example, one or more of the cartilage surface and the bone surface of the patient's femur.

The joint-facing surface of a femoral implant component includes bearing surfaces that contact one or more opposing surfaces during proper joint function. In a total knee implant, the bearing surfaces include one or more portions of the medial and lateral condyles of the femoral component and also the corresponding surfaces on the tibial component. Bearing surfaces also can include the trochlear area of a femoral implant component and the corresponding surface of a patella or patella implant component. In certain embodiments, the femoral implant component can be designed and/or selected to include a joint-facing surface that substantially negatively matches one or more dimensions of an opposing surface, such as a tibial surface or a patellar surface, of the patient's biological structure or of another implant component, such as a tibial implant component or a patellar implant component.

The primary load bearing surfaces of the femoral implant component include the joint-facing surfaces of the medial and lateral femoral condyles. In particular, these condylar surfaces engage the tibia or a tibial implant component during knee joint motion.

In certain embodiments, one or more dimensions or aspects of one or both implant component condyles is designed and/or selected preoperatively to be patient-specific (e.g., to substantially match the patient's condyle or condyles in one or more dimensions or aspects). In addition or alternatively, one or more dimensions or aspects of one or both implant component condyles can be designed and/or selected preoperatively to be patient-engineered (e.g., engineered from patient-specific data to provide to the patient and optimized fit). The patient-specific data used to design and/or select the patient-adapted (e.g., patient-specific or patient-engineered) dimensions or aspects can include one or more images of, at least in part, one or both of the patient's femoral condyles. Accordingly, the manufactured femoral implant component generated from this design and/or selection includes a condyle having one or more patient-adapted dimensions or aspects.

The one or more patient-adapted dimensions or aspects of one or both condyles can include, for example, width in one or more locations, height in one or more locations, intercondylar distance in one or more locations, and one or more curvatures of at least a portion of the joint-facing surface of the condyle. The curvatures can include, for example, one or more of a sagittal curvature on the medial condyle, a coronal curvature on the lateral condyle, a sagittal curvature on the lateral condyle and a coronal curvature on the lateral condyle. The dimensions or aspects on the medial and lateral condyles of the implant component can be designed and/or selected independently of the corresponding dimensions on the other condyle, provided that patient-specific data (e.g., image data) is available for the appropriate condyle.

Any implant component dimensions or aspects that are not patient-adapted can have a standard dimension or aspect. In addition, an implant design can include applying a standard dimension or aspect if patient-specific data exceeds or fails to meet a certain threshold value. For example, in certain embodiments, intercondylar distance can be patient specific; however, if the patient-specific data shows that the patient's intercondylar distance does not exceed a minimum intercondylar distance, such as 40 mm, a standard intercondylar distance of 40 mm can be included in the femoral implant component.

In certain embodiments, one or more of the condylar width, area, and height of an implant component can be designed and/or selected preoperatively to be patient-specific in one or more locations along one or both condyles.

For example, the width across the bone-facing surface of one or both implant component condyles can be designed and/or selected from patient-specific data to substantially match the corresponding width of the patient's resected condyle surface. Similarly, the two-dimensional area of a portion of the bone-facing surface of one or both implant component condyles can be designed and/or selected from patient-specific data to substantially match the corresponding area of the patient's resected condyle. In this way, the implant component can include one or both condyles that, at least in part, substantially covers the width and/or area of resected bone surface on the condyle, for example, by covering up to 100% and at least 90%; greater than 90%; at least 95%; greater than 95%; at least 97%; greater than 97%; at least 98%; greater than 98%; at least 99%; and/or greater than 99% of the width and/or area of the patient's resected condyle surface.

In addition or alternatively, the height of a portion of one or both implant component condyles can be designed and/or selected from patient-specific data to substantially match the corresponding height of the patient's condyle, for example, the height of the corresponding resected portion of the patients' condyle.

In certain embodiments, one or both of the intercondylar distance and the intercondylar angle of the implant component can be designed and/or selected preoperatively to be patient-specific in one or more locations. For example, the distance and/or angle between the implant component condyles can be designed and/or selected from patient-specific data to substantially match the corresponding distance and/or angle of the patient's condyles at one or more locations.

The intercondylar distance can be measured from any point on the medial condyle to any point on the lateral condyle. For example, the intercondylar distance can be measured as the distance between the outside edges of the condyles (e.g., from the medial edge of the medial condyle to lateral edge of the lateral condyle), as the distance between the inside edges of the condyles (e.g., from the lateral edge of the medial condyle to the medial edge of the lateral condyle), or as the distance from the sagittal crest of the medial condyle (i.e., the J-curve of the medial condyle) to the sagittal crest of the lateral condyle (i.e., the J-curve of the lateral condyle).

The intercondylar angle can be measured as the angle between a line on the medial condyle and a line on the lateral condyle. For example, the intercondylar angle can be measured as the angle between a tangent line to any point on the medial condyle to a tangent line to any point on the lateral condyle. One or each tangent line can be defined by a point on the outside edge of a respective condyle, by a point on the inside edge of a respective condyle, or by a point on the J-curve of a respective condyle. Alternatively, one or both lines used to measure the angle can be defined by any two or more points on the same condyle, rather than as a tangent line. In particular, in certain embodiments, the implant component includes a patient-adapted (e.g., patient-specific or patient-adapted) intercondylar angle at the trochlear notch.

In certain embodiments, the joint-facing surface of a femoral implant component can be designed and/or selected to include one or more of a patient-specific curvature, at least in part, a patient-engineered curvature, at least in part, and a standard curvature, at least in part. Various exemplary combinations of implant components having patient-adapted (e.g., patient-specific or patient-engineered) and standard coronal and sagittal condylar curvatures are shown in **Table 4.**

**Table 4: Exemplary combinations of patient-adapted and standard condylar curvatures for a femoral implant component**

| **Group description** | **Medial condyle coronal curvature** | **Medial condyle sagittal curvature** | **Lateral condyle coronal curvature** | **Lateral condyle sagittal curvature** |
|---|---|---|---|---|
| **All standard curvatures** | standard | standard | standard | standard |
| **1 patient-adapted curvature, at least in part** | patient-adapted | standard | standard | standard |
| | standard | patient-adapted | standard | standard |
| | standard | standard | patient-adapted | standard |
| | standard | standard | standard | patient-adapted |
| **2 patient-adapted curvatures, at least in part** | patient-adapted | patient-adapted | standard | standard |
| | patient-adapted | standard | patient-adapted | standard |
| | patient-adapted | standard | standard | patient-adapted |
| | standard | patient-adapted | patient-adapted | standard |
| | standard | patient-adapted | standard | patient-adapted |
| | standard | patient-adapted | standard | patient-adapted |
| | standard | standard | patient-adapted | patient-adapted |
| **3 patient-adapted curvatures, at least in part** | patient-adapted | patient-adapted | patient-adapted | standard |
| | patient-adapted | patient-adapted | standard | patient-adapted |
| | patient-adapted | standard | patient-adapted | patient-adapted |
| | standard | patient-adapted | patient-adapted | patient-adapted |
| **4 patient-adapted curvatures, at least in part** | patient-adapted | patient-adapted | patient-adapted | patient-adapted |

In certain embodiments the joint-facing surface of the femoral implant component can be designed and/or selected to include a patient-specific curvature, at least in part. For example, any one or more of a coronal curvature of the medial condyle, a sagittal curvature of the medial condyle, a coronal curvature of the lateral condyle, and a sagittal curvature of the lateral condyle can be designed and/or selected preoperatively to substantially match the patient's corresponding curvature, at least in part.

In certain embodiments, a load-bearing portion of one or both implant condyles (e.g., from the distal portion to the posterior portion of the implant component's joint-facing condyle surfaces) is designed and/or selected to include one or more patient-specific curvatures. FIGS. 8-1A and 8-1B show the load bearing surfaces of a femoral implant component in coronal view (FIG. 8-1A) and in sagittal view (FIG. 8-1B). As indicated by the figures, the load bearing surface on each of the medial and lateral condyles includes a coronal curvature 1210 and a sagittal curvature 1220. Non-patient-specific curvatures can include standard curvatures (e.g., selected from a family of standard curvatures, for example, a family of 3, 4, 5, 6, 7, or more standard curvatures) and/or engineered curvatures (e.g., engineered from patient-specific data to optimize one or more parameters, for example, as described above). For example, a femoral implant component can include a medial condyle having a patient-specific sagittal curvature, at least in part, and a standard coronal curvature; and a lateral condyle having standard coronal and sagittal curvatures.

In certain embodiments, the joint-facing surface of a femoral implant component can be designed and/or selected to include, at least in part, a patient-engineered curvature. For example, the joint-facing surface can be designed and/or selected to include a patient-engineered curvature, at least in part, for any one or more of the medial condyle coronal curvature, the medial condyle sagittal curvature, the lateral condyle coronal curvature, and the lateral condyle sagittal curvature. In certain embodiments, one or more load-bearing portions of the condylar sagittal and/or coronal curvature is designed and/or selected to be patient-engineered. Non-patient-engineered curvatures can include standard curvatures and/or patient-specific curvatures.

In certain embodiments, an implant curvature can be preoperatively engineered based on patient-specific data to correct or optimize the patient's condyle curvature. The procedure can use a model or a mathematical formula to engineer a corrected or optimized condyle curvature and/or shape. The model or mathematical formula can be based on one or more patient-specific dimensions. For example, in certain embodiments, the implant component can include one or more patient-specific curvatures that have been smoothed to address minor imperfections in the patient's corresponding curvature. In certain embodiments, one condyle can be engineered to have a curvature relative to the corresponding curvature in the patient's other condyle. For example, an implant component can be engineered to have a lateral J-curve engineered based on the patient's medial J-curve. The lateral J-curve can be engineered to have one or more tangent radii that are shorter, for example, 5%, 10%, 15%, 20%, 10-15%, and/or 0-20% shorter, than the corresponding tangent radii of the patient's medial J-curve. In certain embodiments, the implant component can be designed to have a lateral J-curve shape that substantially positively-matches the patient's medial J-curve shape.

In patients in need of knee replacement, the lateral condyle is sometimes deformed or hypoplastic, which can contribute to a valgus deformity. In fact, hypoplastic lateral condyles may be present in 20% of patients that require knee replacement. An implant that is engineered from patient-specific data to address this deformity, by correcting or optimizing the lateral condyle, can include one or more expanded curvatures in one or more locations on the lateral condyle, relative to the patient's corresponding uncut medial or lateral condyle. Accordingly, the implant is engineered to include additional material on the outer, joint-facing surface of the implant component's lateral condyle. The expanded curvature(s) and/or material on the outside of the condyle can be used to design a material savings on the inside of the corresponding section of the implant component, thus maintaining a minimum material / implant thickness. In this way, by adding material to the external contour of the implant component, a minimum material thickness of the implant component can be achieved.

This allows for less material on the inner, bone-facing surface of the implant and, accordingly, less bone to be resected from the corresponding condyle of the patient. Accordingly, this approach uses the patient-adapted design of the implant component to both correct a condyle shape abnormality, such as a lateral condyle abnormality, such as hypoplasia, and to maximize bone preservation.

In certain embodiments, one or more curvatures on one or both condyles can be engineered from patient-specific data, for example, in order to optimize joint kinematics. For example, the medial condyle can be engineered to be 5 mm lateral, which can help lateralize the patella.

Different curvatures can be selected on the medial condyle and the lateral condyle of an implant component. Moreover, one or more curvatures of one condyle can be patient-specific, in whole or in part, while one or more curvatures on the same condyle or on the other condyle can be patient-engineered, in whole or in part, or standard, in whole or in part.

The one or more patient-adapted (i.e., patient-specific or patient-engineered) curvatures and standard curvatures on the joint-facing surface of a femoral implant component can be combined in a condyle to take on any overall shape. FIGS. 8-1C through 8-1G illustrate exemplary curvatures 880 for one or more condylar coronal or sagittal curvatures. An implant component condyle can include a surface curvature corresponding to a section of any one or more geometric shapes, such as a circle, a parabola, a hyperbola, an ellipsoid, and any other geometric shape. The curvature also can include, in part, a straight line portion, as illustrated by the curvature 880 in FIG. 8-1G. Different portions of a condyle, such as the anterior condyle portion, the distal weight bearing portion, and the posterior condyle portion, each can include a different curvature than one or more other portions. For example, in certain embodiments the load bearing portion of the medial condyle can include a different curvature than the non-load bearing sections of the same condyle or any one or more sections of the lateral condyle.

The curvature 880 in FIG. 8-1C corresponds to a section of a circle, and, accordingly, has tangent radii of the same length across its entire curvature. However, the curvatures 880 in FIGS. 8-1D through 8-1G each correspond to a section of a non-circular geometric shape and therefore each include tangent radii of different lengths across their curvatures. Exemplary tangent radii are shown as dotted lines in FIGS. 8-1C through 8-1G.

In certain embodiments, a curvature on the joint-facing surface of a femoral implant component, such as a condylar coronal or sagittal curvature, can include a combination of patient-specific tangent radii, patient-engineered tangent radii, and/or standard tangent radii. In other words, a curvature can include a portion that is patient-specific, a portion that is patient-engineered, and/or a portion that is standard. For example, FIG. 8-1H illustrates a design for a femoral implant component having a J-curve that is partially patient-specific and partially patient-engineered. Specifically, as shown in the figure, the J-curve is designed to be patient-specific except for a distal portion and a posterior portion of the curvature. In the distal portion, the J-curve is smoothed relative to the patient's corresponding J-curve. In the posterior portion, the J-curve is tapered out 1-2 mm relative to the patient's corresponding J-curve, in order to match the height of the patient's remaining posterior cartilage.

Similarly, FIG. 8-1I illustrates two femoral implant components, one having a J-curve that is substantially patient-specific and one having a J-curve that is partially patient-specific and partially patient-engineered. Specifically, both implant components shown on the right side of the figure were designed based on patient-specific data. However, the top implant component includes a J-curve that, at least in its load-bearing portion, substantially positively-matches the patient's J-curve. The bottom implant component includes a J-curve that was engineered in region 890 from patient-specific data.

One or more tangent radii of a condylar curvature of a femoral implant component can be engineered from patient-data to optimize or correct any one or more parameters, for example, any one or more parameters described above. For example, with reference to FIG. 8-1I, the distal load-bearing portion of the J-curve frequently has the flattest surface 892. In certain embodiments, this portion of the J-curve can be engineered to shift the flattest portion anteriorly or posteriorly to correct or optimize the patient's biomechanics and/or alignment. In FIG. 8-1I, the flattest portion 892 of the J-curve in the top implant component appears at the distal portion of the implant. However, the bottom implant is engineered to include the flattest portion 893 of the J-curve further posterior to the distal position. In certain embodiments, an implant component's entire condylar sagittal curvature can be flexed (e.g., rotated anteriorly or posteriorly, for example, about the femoral mechanical axis) in an implant relative to the patient's corresponding curvature, in order to correct or optimize the patient's biomechanics and/or alignment.

In preferred embodiments, the femoral implant component is preoperatively designed and/or selected to include, and the corresponding implant does include, one or both condylar, bearing surfaces having a sagittal curvature (e.g., a J-curve) that, at least in part, substantially positively-matches the corresponding sagittal curvature of the patient's condyle (as determined from patient-specific data including, for example, image data). In addition, the coronal curvature of the implant component can include a standard curvature, for example, selected by choosing from among a family of 5, 6, 7, 8, 9, or 10 implants or implant blanks the one that includes the most similar coronal curvature as compared to the patient's coronal curvature, in one or more locations on the curvature. Alternatively, an implant component condyle curvature can be selected by choosing from among a family of implants or implant blanks the one that, as compared to the patient's curvature, includes longer and/or shorter tangent radii at a load bearing location of the curvature, in order to achieve a less constraining or a more constraining biomechanical situation during knee motion. FIG. 8-1J illustrates the use of a coronal curvature having longer tangent radii (e.g., 40 mm tangent radii) versus a coronal curvature having shorter tangent radii (e.g., 20 mm tangent radii).

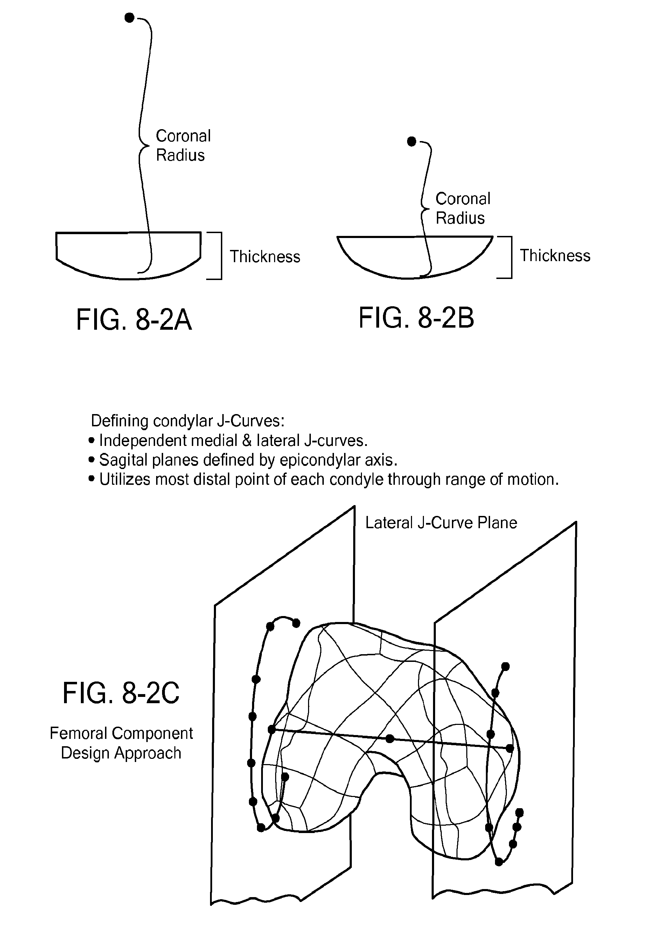

The tangent radii of a human femoral condyle coronal curvature typically range from 20 to 30 mm. In certain embodiments, the coronal radius of one or both condyles of a femoral implant component can, at least in part, correspond to a section of a circle having a tangent radius less than 20 mm, less than 15 mm, 15 mm, greater than 30 mm, 35 mm, greater than 35 mm, 40 mm, and/or greater than 40 mm. FIGS. 8-2A and 8-2B show cross-sections from a coronal view of two femoral condyle sections of a femoral implant component, which corresponds to a section of a circle. As shown, the component cross-sections have the same maximum thickness, but the component in FIG. 8-2A has a longer tangent radius than the component in FIG. 8-2B. As can be seen from the figures, where the width and maximum thickness are the same for the two components, the component with the longer tangent radius allows for more material at the edge of the component, and therefore can be less likely to fail in this area of the femoral implant component. In certain embodiments, the one or more condylar curvatures, e.g., one or more coronal curvatures and/or sagittal curvatures, can be engineered from patient-specific data to be as close to the patient-specific curvature as possible after a threshold minimum thickness and/or minimum edge thickness is achieved.

Various dimensions and aspects of a patient's condyles, such as width, area, height, intercondylar distance, intercondylar angle, and surface curvatures, can be assessed from one or more images of the patient's knee joint. In certain embodiments, a dimension or aspect of the patient's condyles can be assessed to include cartilage on the patient's distal femur. In certain embodiments, a dimension or aspect can be assessed based on the patient's bone, for example, subchondral bone, on the patient's distal femur. If subchondral bone is used to assess the patient's condylar curvature, a standard cartilage thickness (e.g., 2 mm), or an approximate cartilage thickness derived from patient-specific data (e.g., age, joint-size, contralateral joint measurements, etc.) can be used as part of the preoperative implant design, for example, to correct or address for joint-line movement associated with lost cartilage.

The sagittal and/or coronal curvatures of a patient's condyle can be determined from image data by assessing the surface of the condyle in a substantially sagittal or coronal plane. For example, as shown in FIG. 8-2C, a J-curve can be determined independently for lateral and medial condyles. Specifically, using a model of the patient's distal femur, sagittal planes can be determined perpendicular to the patient's epicondylar axis. The most distal points (i.e., the crest) of each condyle can be applied to a sagittal plane to determine the J-curve.

Patellar revision can be very challenging and bone preservation is preferred in the patella. In certain embodiments, the patellar-facing surface of an implant component can be patient-specific, i.e., designed to match the patient's normal patellar tracking in the trochlear groove. Alternatively, the patellar-facing surface of the implant component can be engineered from patient-specific data to optimize one or more parameters, for example, kinematics between component surfaces. A method for designing a patient-adapted implant to optimize tracking of the patella along the trochlear groove of a femoral implant component is described below in Example 4. Specifically, the exemplary implant design in Example 4 uses a patient-specific sagittal curvature, at least in part, and an engineered coronal curvature, at least in part, to allow the patella component to track properly in the trochlear groove. In certain other embodiments, the coronal curvature additionally can be patient-specific. In certain embodiments, the coronal curvature is patient-specific and the sagittal curvature is standard or engineered.

In certain embodiments, a trochlear groove curvature can include a patient-specific portion and a patient-engineered portion. For example, FIG. 8-3D shows a trochlear J-curve (in the trough of the trochlear groove) derived from patient-specific data and superimposed on the patient's distal femur. As indicated by the figure, an implant's s trochlear J-curve can substantially match the patient's trochlear J-curve through in one or more locations, for example, in the anterior portion of the J-curve. The substantially matching implant curvature can include portions that are smoothed relative to the patient's curvature. At the same time, the implant's s trochlear J-curve can be engineered in another portion of the trochlea, for example, in the distal portion of the trochlea. For example, as shown in the figure, the distal portion of the trochlear J-curve is tapered out 1-2 mm, relative to the patient's trochlear J-curve.

In preferred embodiments, the implant's trochlear groove is slightly larger than the corresponding engaging surface of the patella, and/or slightly larger than the patient's corresponding trochlear groove. For example, in certain embodiments, the implant component includes a trochlear groove coronal curvature that is engineered to be slightly wider and/or deeper than the patient's trochlear groove coronal curvature.

Moreover, the implant component can include a trochlear curvature that is shifted in the medial-lateral dimension or in the anterior-posterior dimension in order to optimize the biomechanical situation during knee motion. For example, as indicated by the figure, the implant's trochlear J-curve can be offset laterally, for example, 2 mm laterally, in order to achieve a less constraining biomechanical situation during knee motion.

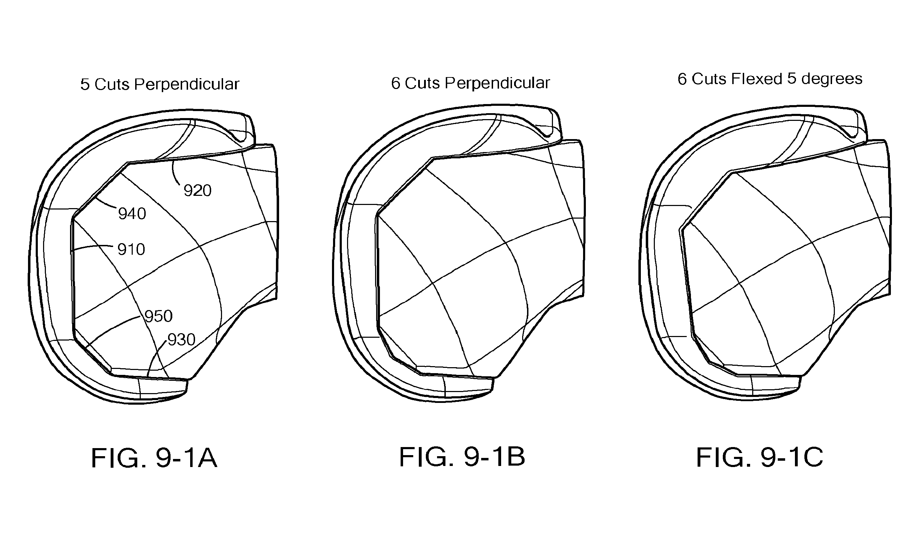

With traditional knee implants the patient's bone is resected to fit the standard shape of the bone-facing surface of the traditional femoral implant component. On its bone-facing surface, the traditional femoral implant component includes five standard bone cuts, as exemplified by the implant shown in FIG. 9-1A. Specifically, a traditional total knee implant includes a distal or horizontal cut 910, an anterior cut 920, a posterior cut 930 along each femoral condyle, an anterior chamfer cut 940, and a posterior chamfer cut 950.

In certain embodiments, one or more portions of the bone-facing surface of the femoral implant are designed based on patient-specific data to substantially negatively-match the uncut surface of the patient's femur, for example, the subchondral bone surface of the femur. In such embodiments, the surgical procedure includes resurfacing (i.e., removing cartilage, at least in part, while substantially retaining the surface of subchondral bone (i.e. cutting away bone), the joint-facing surface of the patient's femur. In certain embodiments, the bone-facing surface(s) of the implant component are designed and/or selected preoperatively, based on patient-specific data, to optimize one or more parameters, such as bone preservation (e.g., minimizing the amount of bone that is resected during the implant procedure).

In certain embodiments one or more portions of the bone-facing surface of the femoral implant includes two or more bone cuts, for example, two, three, four, five, six, seven, eight, nine, or more bone cuts. For example, a femoral implant component can include less than or greater than five bone cuts. In a preferred embodiment, the femoral implant includes six bone cuts, as shown in FIG. 9-1B. In another preferred embodiment, the femoral implant includes seven bone cuts. One or more of the implant bone cuts can be curvilinear or the entire bone-facing surface of the implant component can be curvilinear bone cut.

As exemplified in FIG. 9-1C, in certain embodiments the femoral implant and design can include bone cuts that are rotated or oriented based on a certain flexion angle of the knee, e.g., rotated in the sagittal plane. Any number of bone cuts can be included in an implant device designed with flexed-fit cuts. For example, two, three, four, five, six, seven, eight, nine or more cut planes can be included in a flexed-fit design. One or more of the cuts can be curvilinear or the entire bone-facing surface can be curvilinear. Moreover, any one or more bone cuts can include two or more non-coplanar facets, as described below. The cuts can be oriented at any rotation, for example, at 5, greater than 5, 10, greater than 10, 15, greater than 15, 20, greater than 20, 25, or greater than 25 degrees flexion.

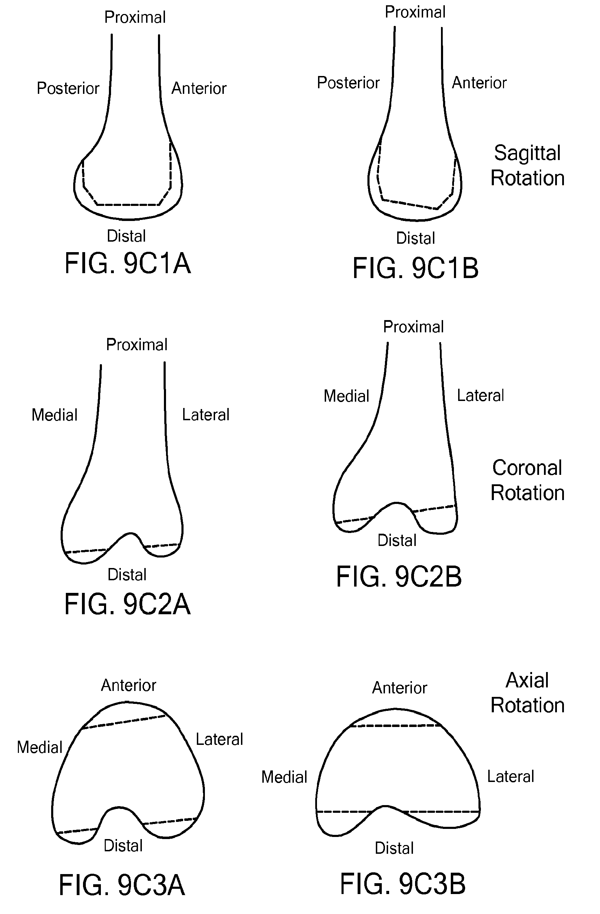

In certain embodiments, the femoral implant component can include one or more bone cuts oriented one or more dimensions, e.g., not only in the sagittal plane, but also in the coronal plane and/or in the axial plane. FIGS. 9C1a through 9C3b show exemplary cross-sections of femoral implant components with bone cuts shown as dashed lines. FIGS. 9C1a and 9C1b show an implant component with traditional bone cuts (FIG. 9C1a) as compared to an implant component with bone cuts rotated in the sagittal plane (FIG. 9C1b). FIGS. 9C2a and 9C2b show an implant component with traditional bone cuts (FIG. 9C2a) as compared to an implant component with bone cuts rotated in the coronal plane (FIG. 9C2b). FIGS. 9C3a and 9C3b show an implant component with traditional bone cuts (FIG. 9C3a) as compared to an implant component with bone cuts rotated in the axial plane (FIG. 9C3b). Such bone cut rotations can help to further optimize bone preservation.

In certain embodiments, two or more trochlear bone cut facets can be substantially tangent to the medial and lateral patellar facets of the patient's uncut bone. In addition or alternatively, two or more trochlear bone cuts can be substantially tangent or parallel to the superior and inferior patellar facets, in particular, when more than two implant trochlear bone cut facets are used. In certain embodiments, one or more trochlear bone cut facets can be curvilinear.

In a traditional femoral implant component, the posterior bone cut includes portions on the medial and lateral condyles that are in the same plane and parallel to each other, and substantially parallel to the anterior cut. However, in certain embodiments described herein, the implant component can include posterior condylar bone cut facets on the medial and lateral condyles, respectively, that are non-coplanar. Alternatively, or additionally, the implant component can include one or more posterior condylar facets that are substantially non-parallel with one or more facts of the anterior bone cut.

In certain preferred embodiments, the posterior condylar bone cut includes a facet on the medial condyle that is substantially perpendicular to the long axis of the medial condyle. Optionally, the facet on the lateral condyle can be perpendicular to the long axis of the lateral condyle. In certain embodiments, the anterior and posterior bone cuts can be substantially non-parallel to the coronal plane in the superoinferior orientation.

In certain embodiments, the posterior bone cut medial and lateral facets of an implant component can be asymmetric with respect to an anterior-posterior (A-P) plane bisecting the implant component. Moreover, one or more posterior bone cut facets can be curvilinear.

In certain embodiments, the distal bone cut of a femoral implant component includes medial and lateral condylar facets that are in the same plane as each other and/or are substantially parallel to each other. The facets can be separated by the intercondylar space and/or by a step cut. In certain embodiments, the implant component can include a distal bone cut having medial and lateral condylar facets that are non-coplanar and/or non-parallel.

In certain embodiments, the distal bone cut or bone cut facets is/are asymmetric with respect to an anterior-posterior (A-P) plane bisecting the implant component. Moreover, the distal bone cut and/or one or more distal bone cut facets can be curvilinear.

Traditional femoral implant components include one anterior chamfer bone cut and one posterior chamfer bone cut. However, in certain embodiments described herein, additional chamfer bone cuts can be included. By increasing the number of chamfer bone cuts on the implant and placing the cuts in close proximity to the tangent of the articular surface, additional bone can be preserved. One or more additional chamfer bone cuts can be substantially tangent to the articular surface. For example, in certain embodiments, the implant component can include one or more additional anterior chamfer cuts and/or one or more additional posterior chamfer cuts.

In certain embodiments, the implant component can include a posterior chamfer bone cut that includes medial and lateral condylar facets that are non-coplanar and/or non-parallel. In certain embodiments, a posterior chamfer bone cut of the implant component can include facets that are asymmetric with respect to an anterior-posterior (A-P) plane bisecting the implant component. Moreover, one or more posterior chamfer bone cuts and/or one or more posterior chamfer bone cut facets can be curvilinear.

In certain embodiments, the implant component can include an anterior chamfer bone cut that includes medial and lateral condylar facets that are non-coplanar and/or non-parallel. In certain embodiments, an anterior chamfer bone cut of the implant component can be asymmetric and/or can include facets that are asymmetric with respect to an anterior-posterior (A-P) plane bisecting the implant component. Moreover, one or more anterior chamfer bone cuts and/or bone cut facets can be curvilinear.

In certain embodiments, the cut plane for one or more bone cuts of the implant component, for example, the distal bone cut and/or one or more of the anterior chamfer bone cuts, can be defined, in part, by the extent of the trochlear gap in the patient's joint. Specifically, one or more of these bone cuts can be designed based on patient-specific data to include a perimeter that matches the contour of the patient's trochlear notch. In certain embodiments, the implant component is patient-adapted so that there is no exposed implant surface on the bone-facing side of the implant component at the trochlear gap. Moreover, one or more of these bone cuts can be designed so that there is no, or minimally exposed, resected bone surface at the trochlear notch.

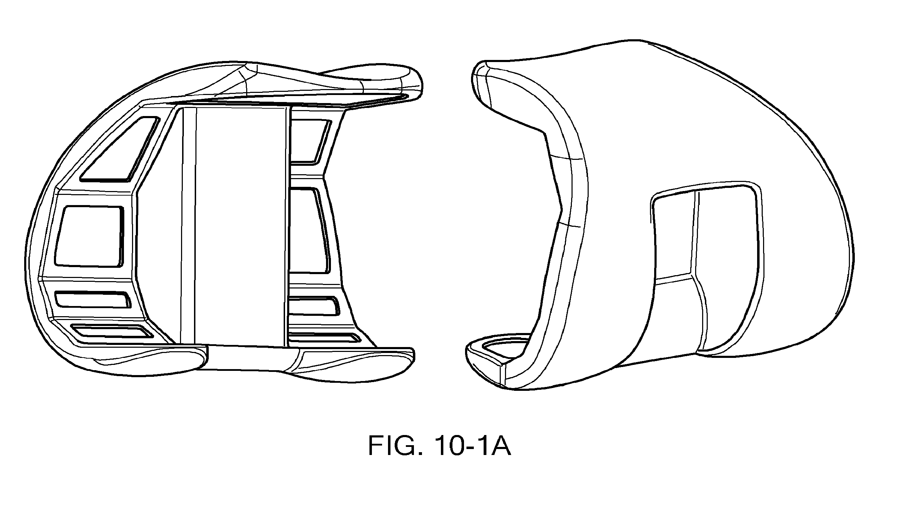

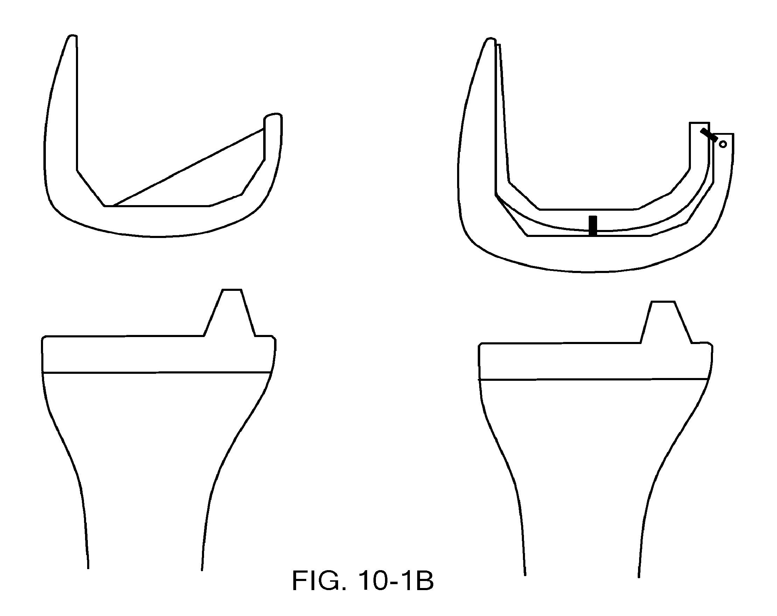

In addition to the implant component features described above, certain embodiments can include features and designs for cruciate substitution. These features and designs can include, for example, an intercondylar housing (sometimes referred to as a "box"), as shown in FIG. 10-1A, a receptacle for a tibial post or projection, and/or one or more intercondylar bars. The intercondylar housing, receptacle, and/or bars can be used in conjunction with a projection or post on a tibial component as a substitute for a patient's posterior cruciate ligament ("PCL"), which may be sacrificed during the implant procedure. Specifically, as shown in FIG. 10-1B, the intercondylar housing, receptacle or bars engage the projection or post on the tibial component to stabilize the joint during flexion, particular during deep flexion.

Different dimensions of the housing, receptacle or bar can be shaped, adapted, or selected based on different patient dimensions and implant dimensions. Examples of different technical implementations are provided in **Table 5.** These examples are in no way meant to be limiting. Someone skilled in the art will recognize other means of shaping, adapting or selecting a housing, receptacle or bar based on the patient's geometry including imaging data.

**Table 5: Examples of different technical implementations of a cruciate-sacrificing femoral implant component**

| **Box, receptacle or bar or space defined by bar and condylar implant walls** | **Patient anatomy, e.g., derived from imaging studies or intraoperative measurements** |
|---|---|
| **Mediolateral width** | Maximum mediolateral width of patient intercondylar notch or fraction thereof |
| **Mediolateral width** | Average mediolateral width of intercondylar notch |
| **Mediolateral width** | Median mediolateral width of intercondylar notch |
| **Mediolateral width** | Mediolateral width of intercondylar notch in select regions, e.g. most inferior zone, most posterior zone, superior one third zone, mid zone, etc. |
| **Superoinferior height** | Maximum superoinferior height of patient intercondylar notch or fraction thereof |
| **Superoinferior height** | Average superoinferior height of intercondylar notch |
| **Superoinferior height** | Median superoinferior height of intercondylar notch |
| **Superoinferior height** | Superoinferior height of intercondylar notch in select regions, e.g. most medial zone, most lateral zone, central zone, etc. |
| **Anteroposterior length** | Maximum anteroposterior length of patient intercondylar notch or fraction thereof |
| **Anteroposterior length** | Average anteroposterior length of intercondylar notch |
| **Anteroposterior length** | Median anteroposterior length of intercondylar notch |
| **Anteroposterior length** | Anteroposterior length of intercondylar notch in select regions, e.g. most anterior zone, most posterior zone, central zone, anterior one third zone, posterior one third zone etc. |

The height or M-L width or A-P length of the intercondylar notch can not only influence the length but also the position or orientation of a bar or the condylar walls.

The dimensions of the housing, receptacle or bar can be shaped, adapted, or selected not only based on different patient dimensions and implant dimensions, but also based on the intended implantation technique, for example intended femoral component flexion or rotation. For example, at least one of an anteroposterior length or superoinferior height can be adjusted if an implant is intended to be implanted in 7 degrees flexion as compared to 0 degrees flexion, reflecting the relative change in patient or trochlear or intercondylar notch or femoral geometry when the femoral component is implanted in flexion.

The internal shape of the housing, receptacle or bar can include one or more planar surfaces that are substantially parallel or perpendicular to one or more anatomical or biomechanical axes or planes. The internal shape of the housing, receptacle, or bar can include one or more planar surfaces that are oblique in one or two or three dimensions. The internal shape of the housing, receptacle, or bar can include one or more curved surfaces that are curved in one or two or three dimensions. The obliquity or curvature can be adapted based on at least one of a patient dimension or a tibial projection or post dimension. The internal surface can be determined based on generic or patient-derived or patient-desired or implant-desired kinematics in one, two, three or more dimensions. The internal surface can mate with a substantially straight tibial projection or post, e.g., in the ML plane. Alternatively, the tibial post or projection can have a curvature or obliquity in one, two or three dimensions, which can optionally be, at least in part, reflected in the internal shape of the box. One or more tibial projection or post dimensions can be matched to, designed to, adapted to, or selected based on one or more patient dimensions or measurements. Any combination of planar and curved surfaces is possible.

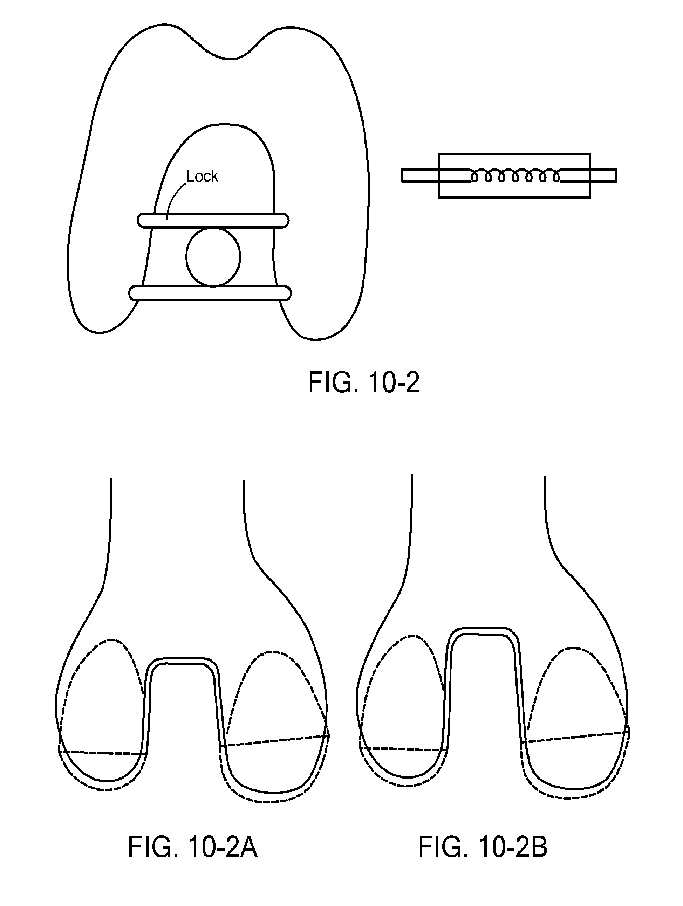

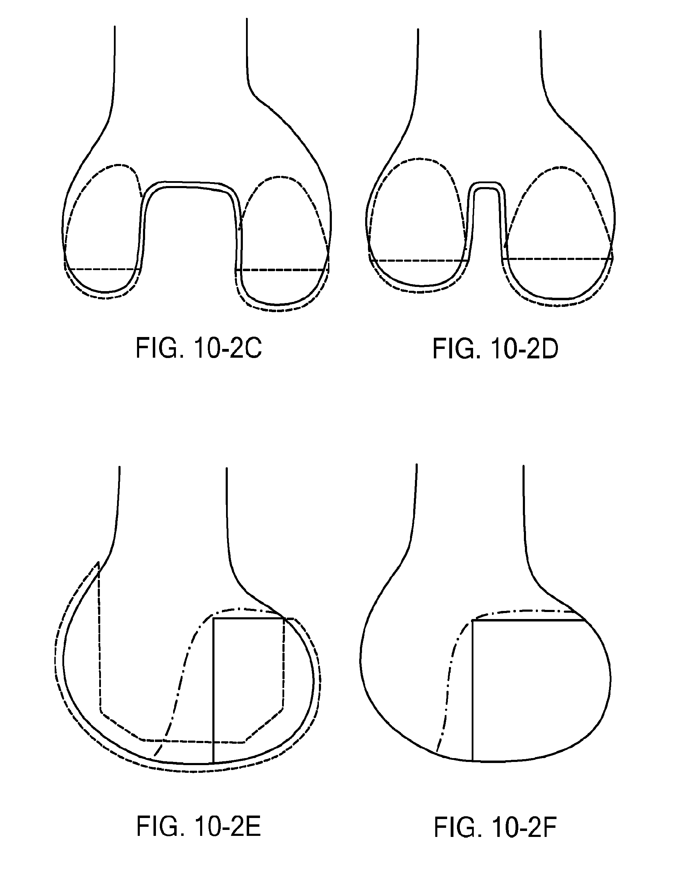

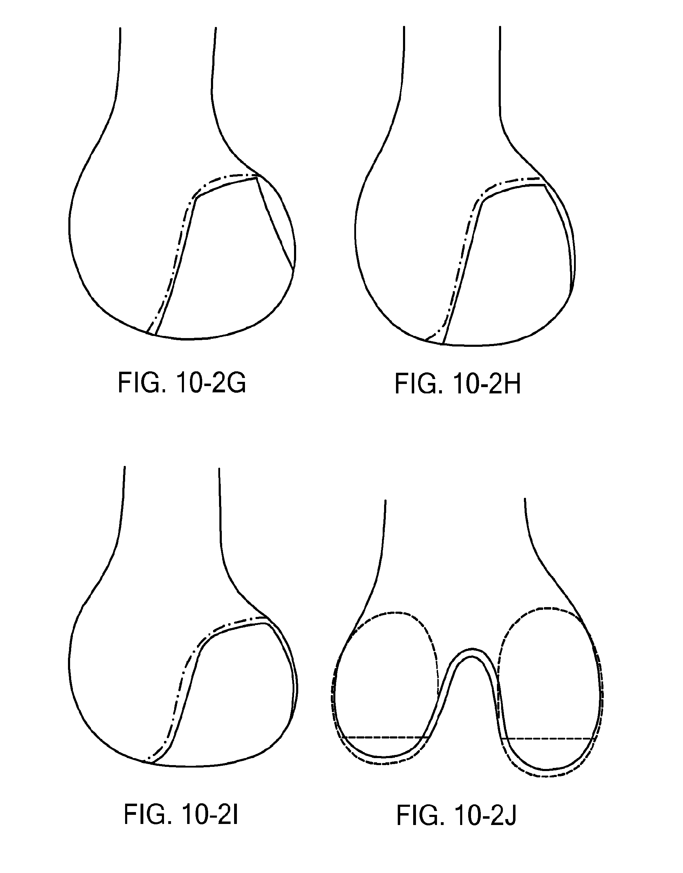

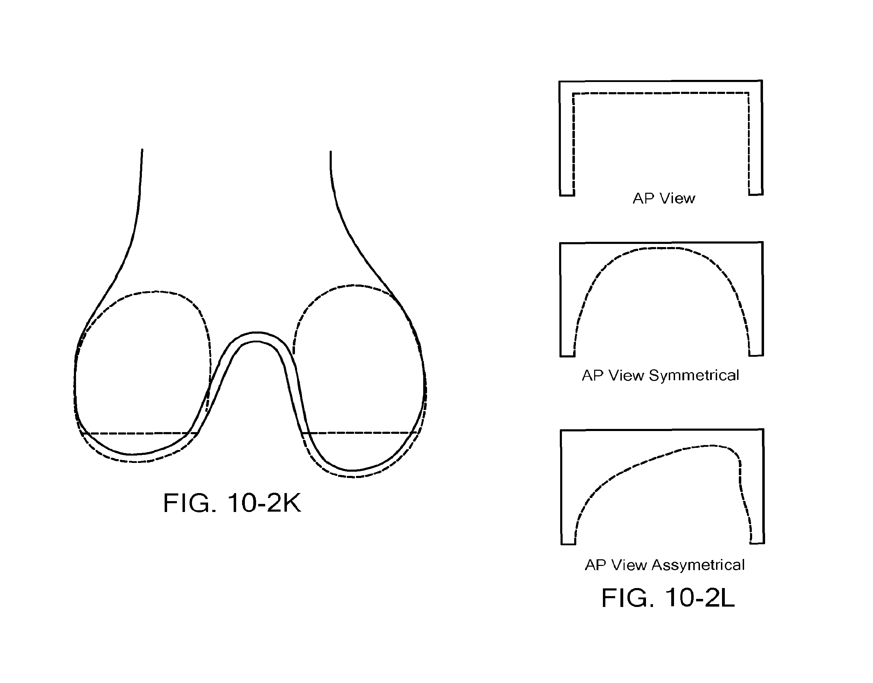

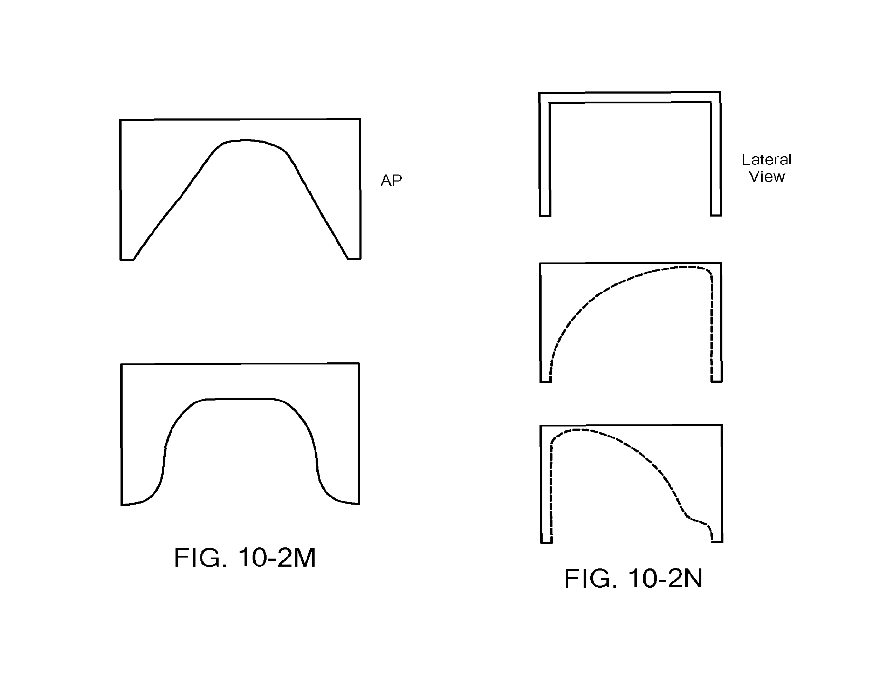

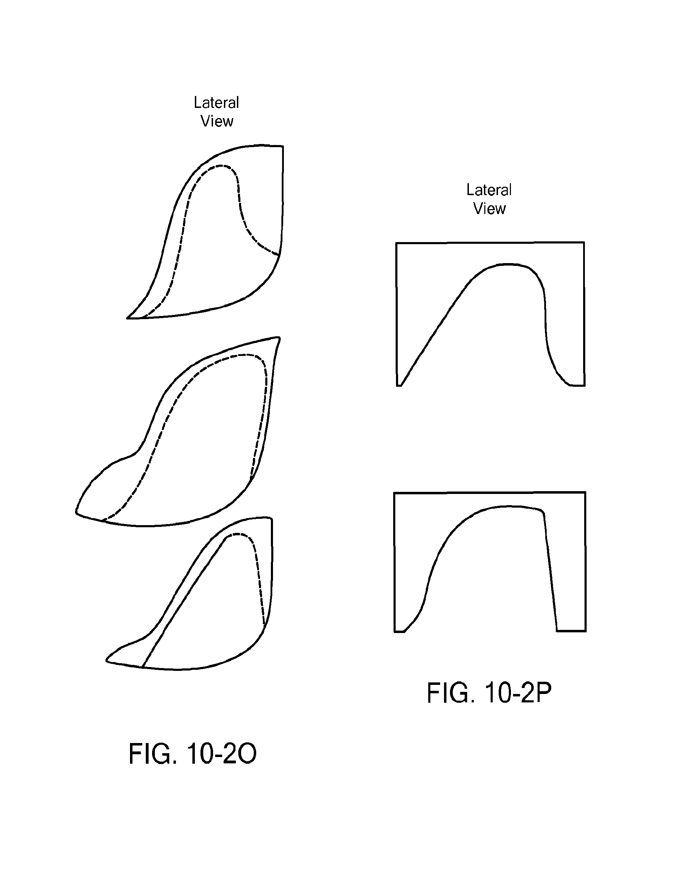

FIGS. 10-2A through 10-2K show various embodiments and aspects of cruciate-sacrificing femoral implant components. FIG. 10-2A shows a box height adapted to superoinferior height of intercondylar notch. The dotted outlines indicate portions of the bearing surface and posterior condylar surface as well as the distal cut of the implant. FIG. 10-2B shows a design in which a higher intercondylar notch space is filled with a higher box or receptacle, for example, for a wide intercondylar notch. FIG. 10-2C shows a design in which a wide intercondylar notch is filled with a wide box or receptacle. The mediolateral width of the box is designed, adapted or selected to the wide intercondylar notch. FIG. 10-2D shows an example of an implant component having a box designed for a narrow intercondylar notch. The mediolateral width of the box is designed, adapted or selected for the narrow intercondylar notch. FIG. 10-2E shows an example of an implant component having a box for a normal size intercondylar notch. The box or receptacle is designed, adapted or selected for its dimensions. (Notch outline: dashed & stippled line; implant outline: dashed lines). FIG. 10-2F shows an example of an implant component for a long intercondylar notch. The box or receptacle is designed, adapted or selected for its dimensions (only box, not entire implant shown). FIG. 10-2G is an example of one or more oblique walls that the box or receptacle can have in order to improve the fit to the intercondylar notch. FIG. 10-2H is an example of a combination of curved and oblique walls that the box or receptacle can have in order to improve the fit to the intercondylar notch. FIG. 10-2I is an example of a curved box design in the A-P direction in order to improve the fit to the intercondylar notch. FIG. 10-2J is an example of a curved design in the M-L direction that the box or receptacle can have in order to improve the fit to the intercondylar notch. Curved designs are possible in any desired direction and in combination with any planar or oblique planar surfaces. FIG. 10-2K is an example of oblique and curved surfaces in order to improve the fit to the intercondylar notch. FIGS. 10-2L through 10-2P show lateral views of different internal surfaces of boxes.

### 3. Guide tools for installing a joint implant

A variety of traditional guide tools are available to assist surgeons in preparing a joint for an implant, for example, for resecting one or more of a patient's biological structures during a joint implant procedure. However, these traditional guide tools typically are not designed to match the shape (contour) of a particular patient's biological structure(s). Moreover, these traditional guide tools typically are not designed to impart patient-optimized placement for the resection cuts. Thus, using and properly aligning traditional guide tools, as well as properly aligning a patient's limb (e.g., in rotational alignment, in varus or valgus alignment, or alignment in another dimension) in order to orient these traditional guide tools, can be an imprecise and complicated part of the implant procedure.

Certain embodiments described herein provide improved surgical guide tools and methods for preparing a patient's biological structure during a joint implant procedure.

### 3.1 Patient-specific guide tools

Certain embodiments a guide tool having at least one patient-specific bone-facing surface that substantially negatively-matches at least a portion of a biological surface at the patient's joint. The guide tool further can include at least one aperture for directing movement of a surgical instrument, for example, a securing pin or a cutting tool. One or more of the apertures can be designed to guide the surgical instrument to deliver a patient-optimized placement for, for example, a securing pin or resection cut. In addition or alternatively, one or more of the apertures can be designed to guide the surgical instrument to deliver a standard placement for, for example, a securing pin or resection cut.

Certain embodiments can include a guide tool that is flexible and includes at least one patient-specific bone-facing surface that substantially negatively-matches at least a portion of a biological surface at the patient's joint. The guide tool's flexibility, in combination with its patient-specific bone-facing surface, can allow it to snap-fit into position onto the patient's biological structure. For example, the guide tool can be snap-fit to a patient's biological structure by applying force to the guide tool to cause it to expand and engage the structure. During or following the expansion and engagement, the tool can be adjusted so that the patient-specific bone-facing surface engages the corresponding surface of the biological structure.

The flexibility of the guide tool can be altered or enhanced using materials and processes known in the art. For example, various types and/or combinations of polymers and polymer manufacturing techniques can be used to make a guide tool with the appropriate flexibility to provide a snap-fit.

The bone-facing surface of the guide tool can be patient-specific (e.g., substantially negatively-match) for one or more surface dimensions or aspects of the patient's biological surface including, for example, width, area, height, distance, angle, and curvature. For example, in certain embodiments, a dimension or aspect of the patient's biological structure can be assessed to include the cartilage on its surface. In certain other embodiments, a dimension or aspect can be assessed based on the patient's bone surface, for example, subchondral bone surface.

If a subchondral bone surface is used to assess the patient's biological surface, a standard cartilage thickness (e.g., 2 mm), or an approximate cartilage thickness derived from patient-specific data (e.g., age, joint-size, contralateral joint measurements, etc.) can be used as part of the design for the guide tool, for example, to design the size and bone-facing surface of the guide tool. The standard or approximate cartilage thickness can vary in thickness across the assessed surface area. In certain embodiments, this design can be used with a similarly designed implant, for example, an implant designed to include a standard or approximate cartilage thickness.

### 3.2 Patient-engineered guide tools

In certain embodiments, a guide tool includes at least one feature for directing a surgical instrument to deliver a patient-engineered feature to the patient's biological structure, for example, a resected hole or a resection cut for engaging a patient-engineered implant peg or a patient-engineered implant bone-facing surface. In addition to the patient-engineered feature, in certain embodiments one or more of the guide tool's bone-facing surfaces can be designed to be patient-specific so that it substantially negatively-matches a portion of the patient's joint surface. In addition or alternatively, one or more of the guide tool's bone-facing surfaces can be standard in shape.

### 3.3 Exemplary guide tool configurations

The guide tools described herein can include any combination of patient-specific features, patient-engineered features, and/or standard features. For example, a patient-specific guide tool includes at least one feature that is preoperatively designed and/or selected to substantially match one or more of the patient's biological features. A patient-engineered guide tool includes at least one feature that is designed or selected based on patient-specific data to optimize one or more of the patient's biological features to meet one or more parameters. A standard guide tool includes at least one feature that is selected from among a family of limited options, for example, selected from among a family of 5, 6, 7, 8, 9, or 10 options. Accordingly, any one guide tool can be both patient-specific in view of its patient-specific features and patient-engineered in view of its patient-engineered features. Such a guide tool also can include standard features as well. **Table 12** describes the various combinations of three features of a single guide tool with regard to being patient-specific features, patient-engineered features, and/or standard features of the exemplary guide tools. Moreover, in certain embodiments a set or kit of guide tools is provided in which certain guide tools in the set or kit include patient-specific, patient-engineered, and/or standard features. For example, a set or kit of guide tools can include any two or more guide tools described in **Table 6.**

**Table 6: Patient-specific, patient-engineered, and standard features of exemplary guide tools**

| **Exemplary Guide tool** | **Feature #1** | **Feature #2** | **Feature #3** |
|---|---|---|---|
| **A guide tool that includes at least 3 PS features** | P | P | P |
| **A guide tool that includes at least 3 PE features** | PE | PE | PE |
| **A guide tool that includes at least 3 S features** | St | St | St |
| **A guide tool that includes at least 2 PS features and at least 1 PE feature** | P | P | PE |
| | P | PE | P |
| | PE | P | P |
| **A guide tool that includes at least 2 PS features and at least 1 S feature** | P | P | St |
| | P | St | P |
| | St | P | P |
| **A guide tool that includes at least 1PS feature and at least 2 PE features** | PE | PE | P |
| | PE | P | PE |
| | P | PE | PE |
| **A guide tool that includes at least 1PS feature and at least 2 S feature** | St | St | P |
| | St | P | St |
| | P | St | St |
| **A guide tool that includes at least 2PE features and at least 1 S feature** | PE | PE | St |
| | PE | St | PE |
| | St | PE | PE |
| **A guide tool that includes at least 1PE feature and at least 2 S features** | St | St | PE |
| | St | PE | St |
| | PE | St | St |
| **A guide tool that includes at least 1PS feature, at least 1 PE feature, and at least 1 S feature** | P | PE | St |
| | P | St | PE |
| | PE | P | St |
| | PE | St | P |
| | St | P | PE |
| | St | PE | P |

| | | | |
|---|---|---|---|
| P indicates a patient-specific feature, PE indicates a patient-engineered feature, and St indicates a standard feature | | | |

A guide tool can be used for one or more purposes during an implant procedure. For example, one or more guide tools can be used to establish resected holes in a patient's biological structure, to establish resected cuts in a patient's biological structure, and/or to balance or estimate fit of a joint implant. The following subsections describe exemplary guide tools and guide tool features that can help to establish resected holes, to establish resected cuts, and to balance or estimate fit of a joint implant.

As shown in FIG. **S6-1A,** a guide tool S600 can include one or more apertures S600 for guide a tool for establishing resected holes in a patient's biological structure. One or more of these resected holes can be used to engage a peg on one or more subsequently used guide tools, e.g., guide tools for establishing resection cuts. Specifically, one or more pegs projecting from the bone-facing surface of a subsequently used guide tool can be placed into the resected hole to fix the placement of the subsequently used guide tool. Alternatively or in addition, one or more of these resected holes can be used to a peg on an implant component. Specifically, one or pegs projecting from the bone-facing surface of the implant can be placed into the resected hole to fix the placement of the implant component. In certain embodiments, cement can be applied to the resected hole and/or the implant peg to secure the placement of the implant.

A variety of aperture configurations can be used for the guide tools described herein. In certain embodiments, a guide tool aperture configuration can be patient-engineered to engage a particular patient-engineered peg configuration on the corresponding implant component. Moreover, aperture features such as size, shape, angle and, optionally, stop depth, can be designed to substantially match the peg features for the corresponding implant component. For example, in certain embodiments the aperture cross-section can be round to match a round peg on the implant component. However, in certain embodiments, as suggested by FIGS. 10-3B, the aperture cross-section can include a "+" or cross-like configuration to match the corresponding peg on the implant component.

A variety of aperture sizes can be used. For example, a 7 mm diameter aperture can be used. Moreover, the aperture can be oriented to establish a resected hole at any angle. For example, an aperture can be oriented to establish a resected hole in line with the femoral mechanical axis. Alternatively, an aperture can be oriented to establish a resected hole at an anterior-leaning angle to match the angle of the corresponding implant peg. For example, one or more apertures can be oriented anteriorly to establish a 5 degree, 5-10 degree, 10 degree, 10-15 degree, and/or 15 degree anterior-leaning angle relative to the femoral mechanism axis. The aperture can be oriented to establish a resected hole that is oriented at the same angle or at different angles as one or both of the anterior and posterior cuts of the implant component. In certain embodiments, a depth-stop can be used so that the aperture allows a resected hole having a certain maximum depth.

In certain embodiments, a guide tool includes at least one moveable aperture and, optionally, a moveable corresponding bushing. FIG. S6-2 depicts a guide tool having a moveable lateral aperture and bushing. Specifically, the lateral aperture and bushing in the figure are moveable in the A-P direction. However, a moveable aperture (and, optionally, a moveable bushing) can be moveable in the A-P direction, in the M-L direction, or in all directions from the aperture center (e.g., can be rotated in a circular pattern). Moreover, the guide tool can include two or more moveable apertures. For example, a lateral aperture and a medial aperture, and optionally one or both corresponding bushings, can be moveable. Alternatively, one or more apertures (and optional bushings) can be fixed, for example, as is the medial aperture and bushing in FIG. S6-2.

The guide tool that includes one or more moveable apertures also can include one or more patient-specific features, one or more patient-optimized features, and/or one or more standard features. Moreover, the guide tool can be used with a subsequently used guide tool or implant that includes one or more patient-specific features, one or more patient-optimized features, and/or one or more standard features.

A guide tool that includes one or more moveable apertures for establishing resected holes can be used during surgery to adjust the orientation of subsequently placed guide tools and/or implant components. For example, one or more moveable apertures in a femoral guide tool can be used to adjust the femoral flexion, femoral rotation, and/or tibial external rotation of the particular patient's femoral implant.

With reference to the exemplary guide tool shown in FIG. S6-2, a surgeon can snap-fit the guide tool on the femur, observe placement of the moveable aperture as well as other guide tool features relative to one or more biological landmarks, and, if appropriate, move the moveable aperture in order to adjust the orientation of the subsequently installed femoral implant component on the femur. For example, if the surgeon moves the lateral aperture posteriorly to the 3 degree mark, as shown in the figure, the lateral resected hole and engaging lateral peg of the subsequently installed femoral implant component also would be shifted posteriorly. With the medial aperture fixed, this would posteriorly flex the lateral side of the implant component and axially rotate the orientation of the implant component on the femur. As shown in FIG. S6-2, the movable bushing can be limited to one or more ranges of motion and can be accompanied by markings and/or values displayed on the tool surface indicating the rotation in relation to one or more axes. Moreover, in certain embodiments, patient-specific information and/or standard rules can be used to limit the extent of movement of the moveable aperture. For example, the limit of posterior movement of the aperture in the guide tool could be established as the point which still allows a subsequent guide tool fitting into the resected hole established by the aperture to guide a lateral posterior bone resection that removes some minimum amount of bone, for example, so that intersect between the posterior resection cut and posterior chamber cut is below the bone surface.

In certain embodiments, the movable aperture also could allow for a balancing technique. For example, a navigation chip can be placed into the lateral joint in flexion, e.g., as a laminar spreader. This rotates the femur internally and the joint gap can be balanced by moving the lateral aperture, for example, posteriorly. So we have all four techniques integrated into our system and everyone's believe would be satisfied. The surgeon could then decide what he wants right there at surgery.

In certain embodiments, the extent limit of movement of the moveable aperture in the A-P direction can be limited to 3 degrees, less than 3 degrees, 5 degrees, less than 5 degrees, 7 degrees, and/or less than 7 degrees.

In certain embodiments, instead of including a moveable aperture, the guide tool can include one or more alterative apertures and/or an extended opening, for example, and opening that includes the approximate area through which a moveable aperture can move. Then, a bushing (e.g., metal or plastic) or other device can snap, slide, or lock into place onto one of the alternative apertures or at a certain location in the extended opening. For example, the bushing or other device could lock into place at alternative apertures or in the extended opening at 3, 5 and 7 degrees. In this way, the surgeon can alter the implant orientation as he or she deems appropriate. Alternatively, a guide tool can include an aperture bushing that is fixed in one location but can swivel, for example, between 3 and 7 degrees.

The guide tools described herein can include features and apertures for various steps in a joint replacement procedure. For example, in certain embodiments, one or more guide tools can be used to establish all the resection cuts associated with installation of a particular implant component. As noted above, one or more of the guide tools can include one more patient-specific features and/or one or more patient-engineered features and/or one or more standard features.

In certain embodiments, a set of three guide tools can be used to establish all the resection cuts associated with installation of a particular implant component. For example, the three guide tools shown in FIG. S6-1A, S6-1B, and S6-1C can be used together to establish all the resection cuts associated with installation of a femoral implant component. In particular, FIG. S6-1A shows a peg hole driver guide tool for establishing pin or peg holes for subsequent guide tools and, optionally, for the femoral implant component; FIG. S6-1B shows a guide tool for making distal and posterior resection cuts to the distal femur; and FIG. S6-1C shows two options for a third guide tool for making anterior and posterior chamfer cuts. The particular set of guide tools shown in these figures is designed for a tibia first technique; however, the features of the guide tools for preparing a knee joint as described herein can be applied to both a tibia first technique and to a femur first technique.

Furthermore, a spacer, such as a lateral spacer, can be used for balancing to equal distal asymmetry. The spacer can include patient-specific and/or patient-engineered features. A series of balancer chips with thicknesses can be included, for example a series of chips having 1 mm increases in thickness. Alternatively or in addition, a guide tool can include an integrated spacer thickness to allow surgeon to assess tension without needing a spacer. For example, a guide tool for establishing femoral resection cuts can include an integrated space having a tibial resection depth added to the guide tool external surface.

Peg hole driver guide tools can be designed to be patient-specific on their bone-facing surface to allow a snap-fit onto a particular patient's femur. Other features, such as thickness, joint-facing surface, and peg placement and size, also can be patient-specific or patient-engineered. Alternatively or in addition, one or more features can be standard. In the peg hole driver guide tool shown in FIG. S6-1A, the peg size is 6 mm, which can accommodate a ¼ drill size; however, any peg hole size can be used.

Resection cut guide tools can be designed to be patient-specific on their bone-facing surface to allow a snap-fit onto a particular patient's femur. Moreover, other features, such as thickness, joint-facing surface, and peg placement and size, also can be patient-specific or patient-engineered. Alternatively or in addition, one or more features can be standard. The cut guide tool shown in FIG. S6-1B is designed to rest on 2 mm thick cartilage relative to CT data and includes a standard tool thickness of 4 mm; however other standard thicknesses can be used, for example, a 2 mm or a 3 mm thickness. Moreover, the cut guide tool shown in FIG. S6-1B includes three optional pin placements for stabilization and a posterior beam to give stability and rigidity to the tool. The lateral spacer shown on the left side of FIG. S6-1B can rest on cut tibia to approximate the thickness of asymmetry.

The cut tools shown in FIG. S6-1C is designed to follow the cut tool shown in FIG. S6-1B and can complete the remaining chamfer cuts for the distal femur. Each of the cut tools shown in the figure includes two pin placements for stabilization. The tool on the left includes short pegs to be positioned in the peg holes established by a peg hole driver guide tool, for example, as shown in FIG. S6-1A. The tool on the right includes no lateral peg to allow for external rotation of the guide tool to establish rotated chamfer cuts.

In certain embodiments, fewer than three guide tools, for example, two guide tools or one guide tool can be used to establish the peg holes and all the resection cuts associated with installation of a particular implant component. For example, the single guide tool shown in FIG. S6-2A can be used to establish all the resection cuts associated with installation of a femoral implant component, including, for example, a distal cut with one more facets, a posterior cut with one or more facets, an anterior cut, an anterior chamfer cut with one or more facets, one or more posterior chamfer cuts each with one or more facets, and one or more step cuts, for example, between two facets of a cut. The apertures (e.g., holes and slots) in the guide tool and edges of the guide tools can be used to guide peg hole placement and resection cuts. For example, the posterior surface of the guide tool shown in FIG. S6-2A can be used to establish the posterior resection cuts. Optional guide tool attachments can be used to enhance the guidance for one or more of the cutting holes or slots in the cut guide tool. For example, the optional guide tool attachments shown in FIG. S6-2B can be used to enhance one or more cutting or drilling surfaces.

FIG. S6-2C shows an additional embodiment of a single guide tool that can be used to establish the peg holes and all the resection cuts associated with installation of a femoral implant component. In particular, the single guide tool is presented with three different thicknesses, which corresponds to each of images shown on the figure. Having the single guide tool in different thicknesses allows a surgeon to select the optimum thickness for balancing before initiating the resection cuts. The guide tool can come in 2, 3, 4, 5, 6, 7, 8, 9, 10, or more thicknesses. One the surgeon has selected the guide tool with the appropriate thickness, the distal resection cut and all subsequent resection cuts can be made using the selected guide tool. This allows the distal resection cut to be placed at the appropriate position from the joint line. In addition, two of the images in the figure show three optional guide tool attachments for enhancing the cutting or drilling surfaces of the single guide tool. One or more guide tool attachments can be used to enhance the cutting and/or drilling surfaces for one, two, more than two, or all of the apertures in a single guide tool. In this embodiment, numbers are included on the surface of the guide tools to indicate the order of the resection cuts. FIG. S6-2D shows a set of resection cut guide tools that together can be used to facilitate the same peg holes and resection cuts that can be facilitated with the single guide tool shown in FIG. S6-2C. The middle three guide tools (labeled A, B, and C) include the same resection cuts but have different thicknesses to allow for balancing, as described above. This group of guide tools can be included in a kit along with the single guide tool described above, for example, to act as a back-up set of guide tools.

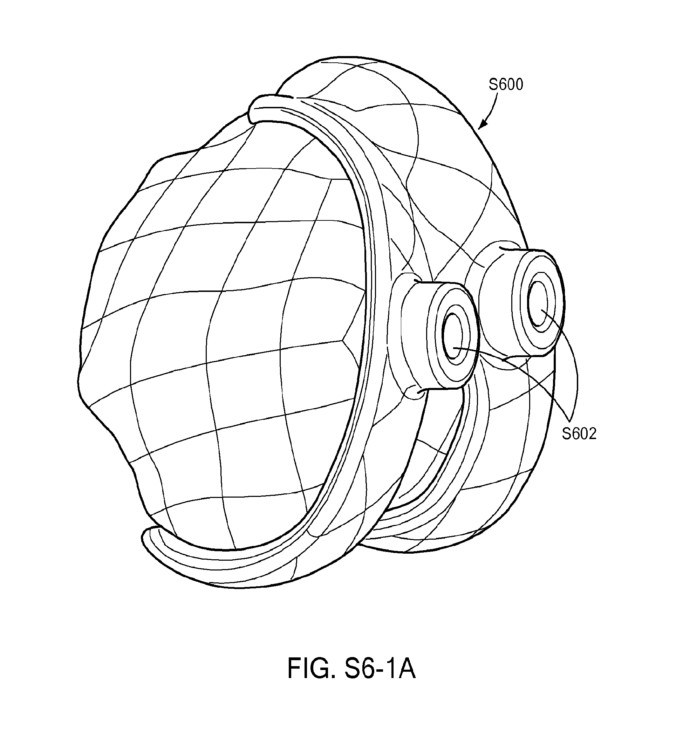

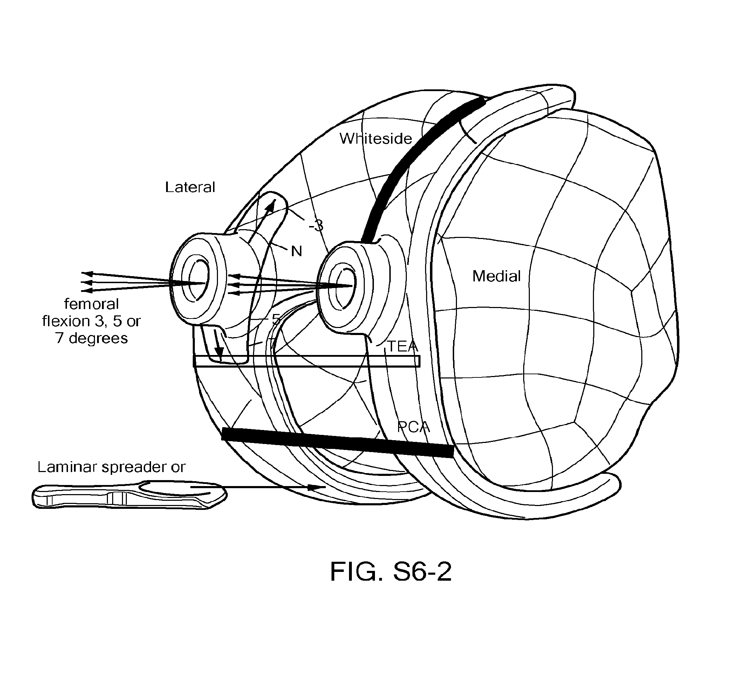

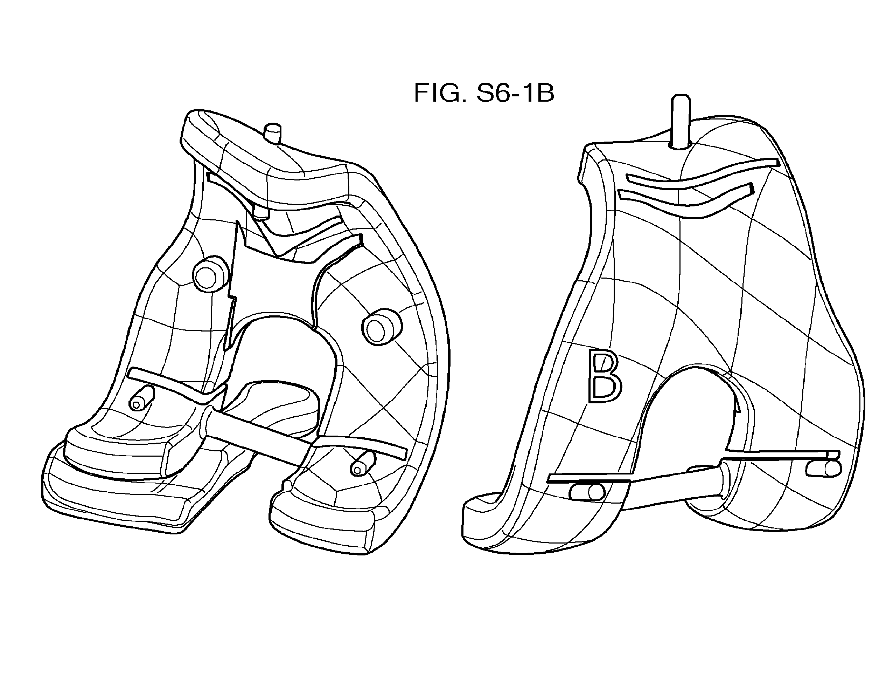

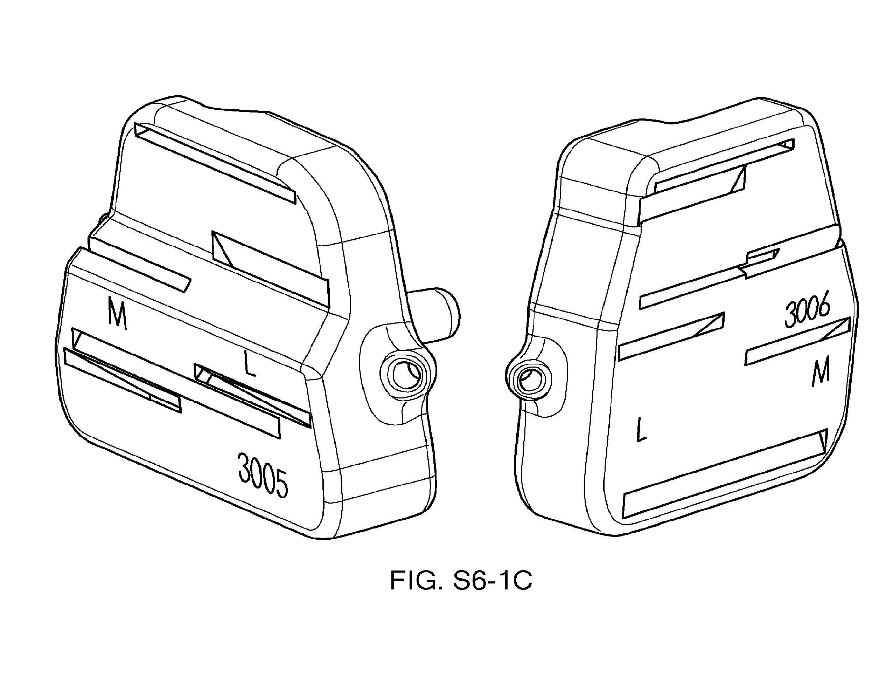

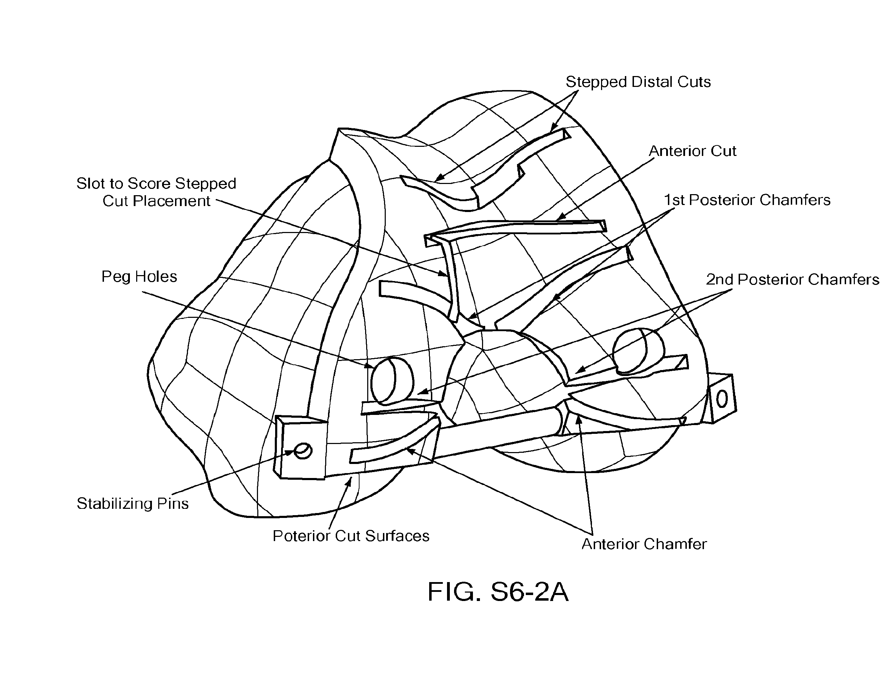

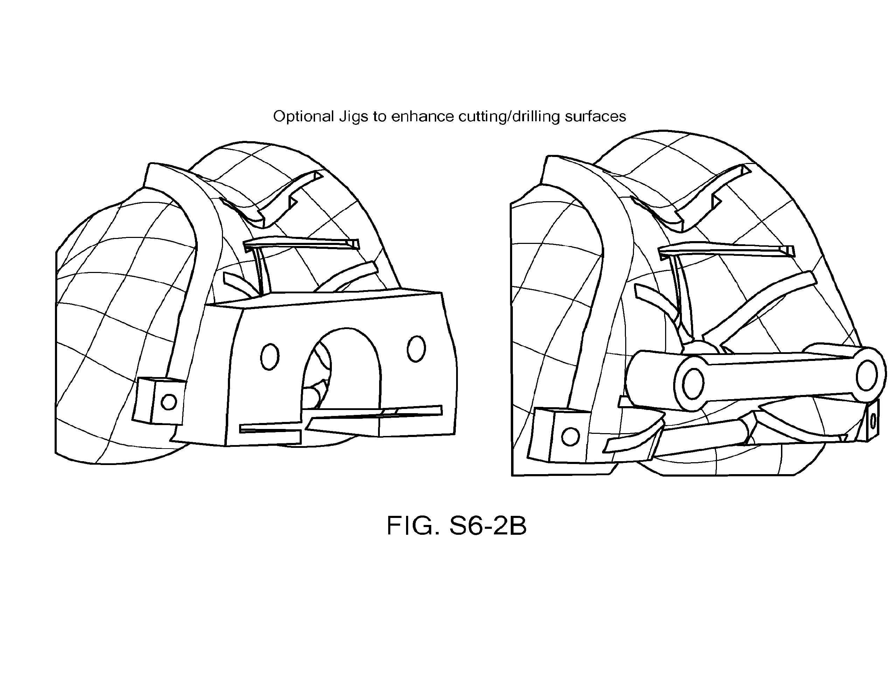

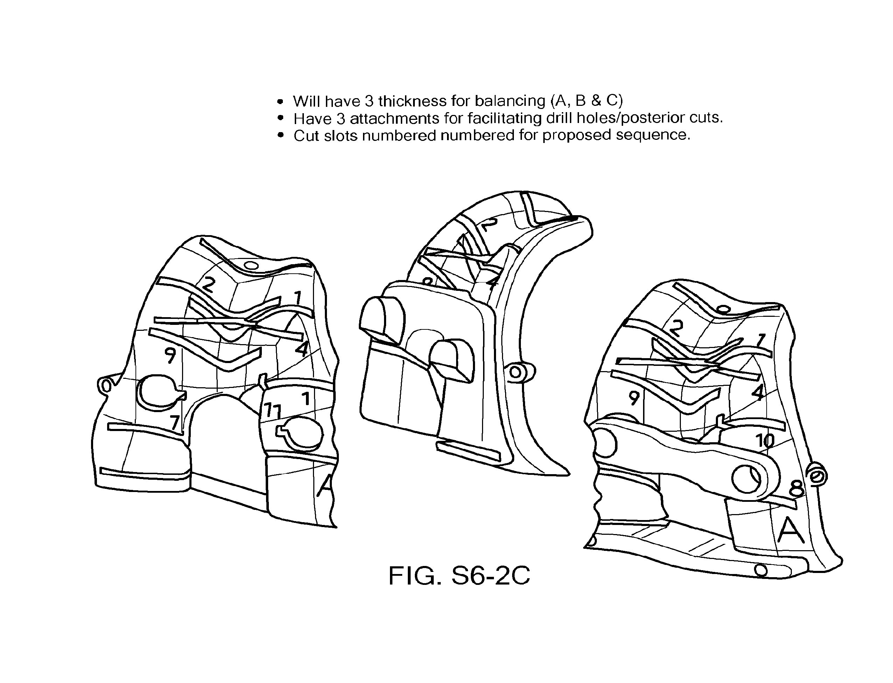

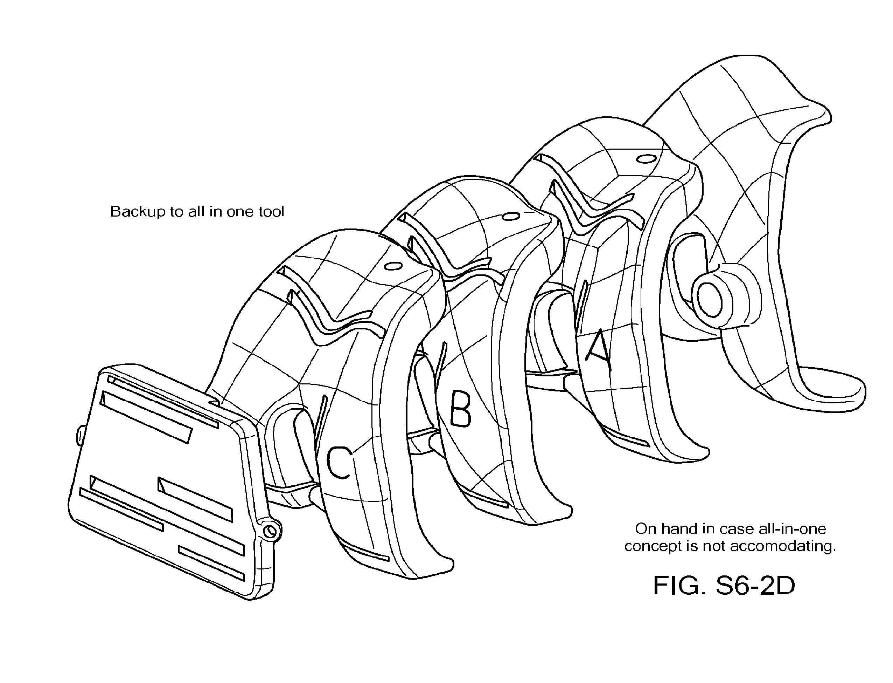

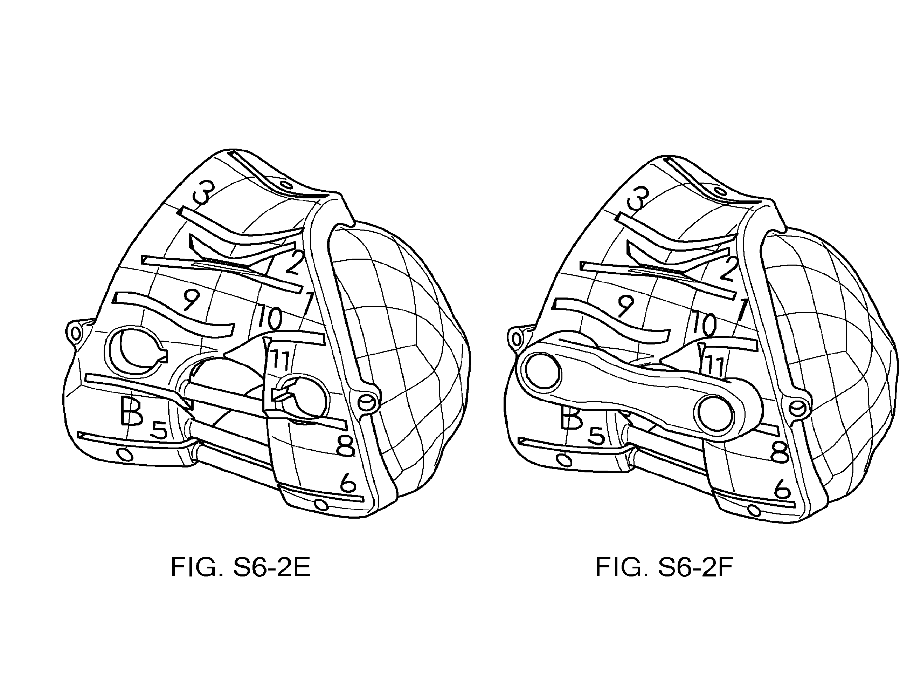

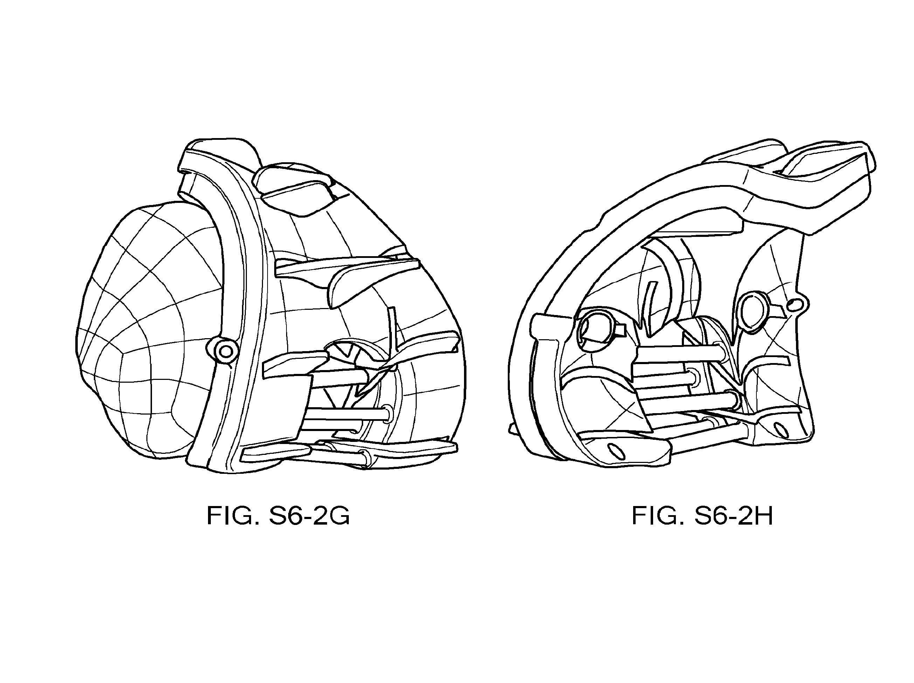

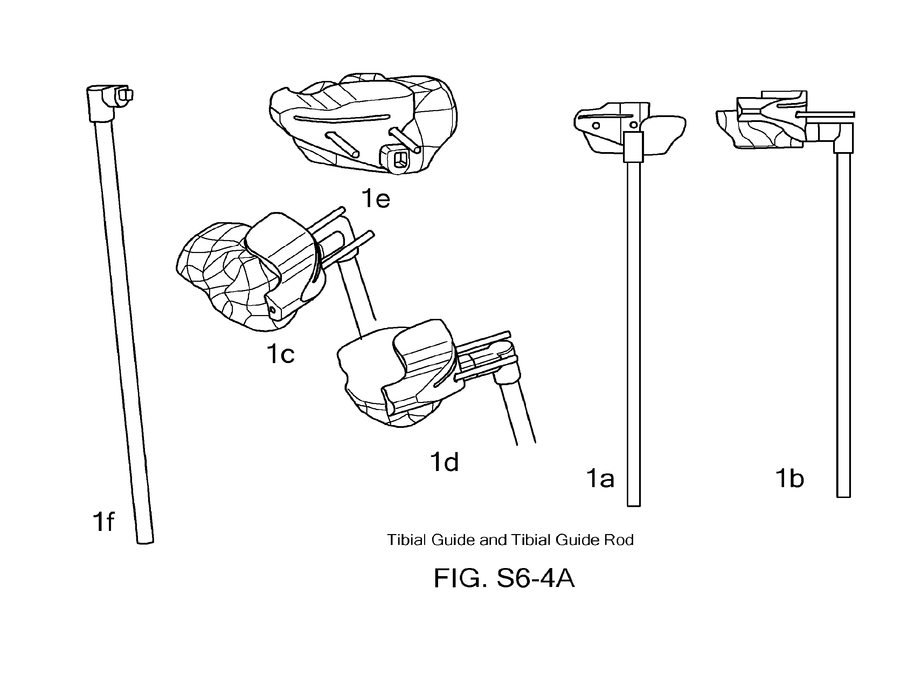

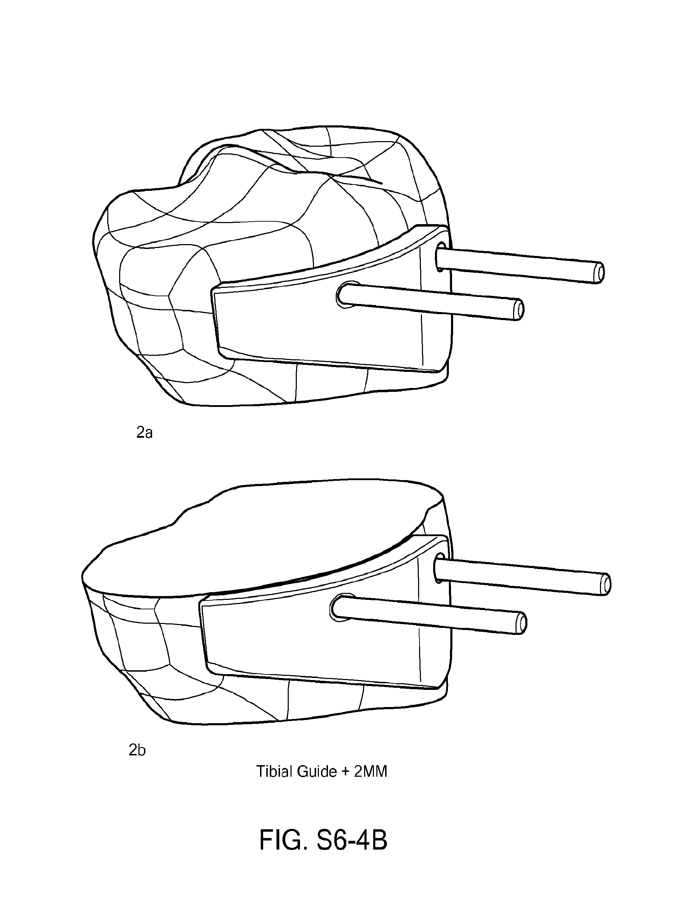

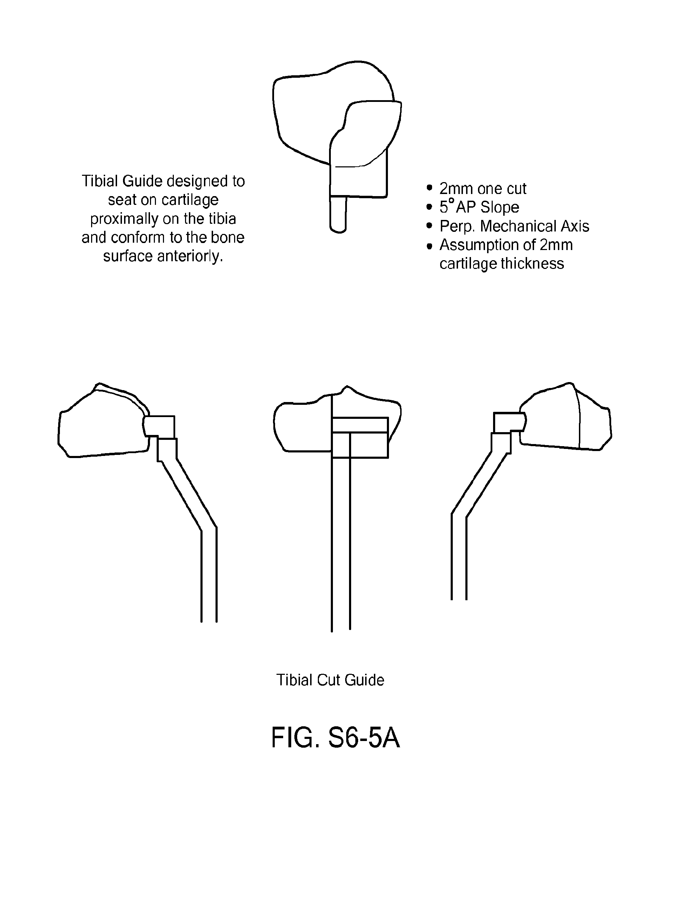

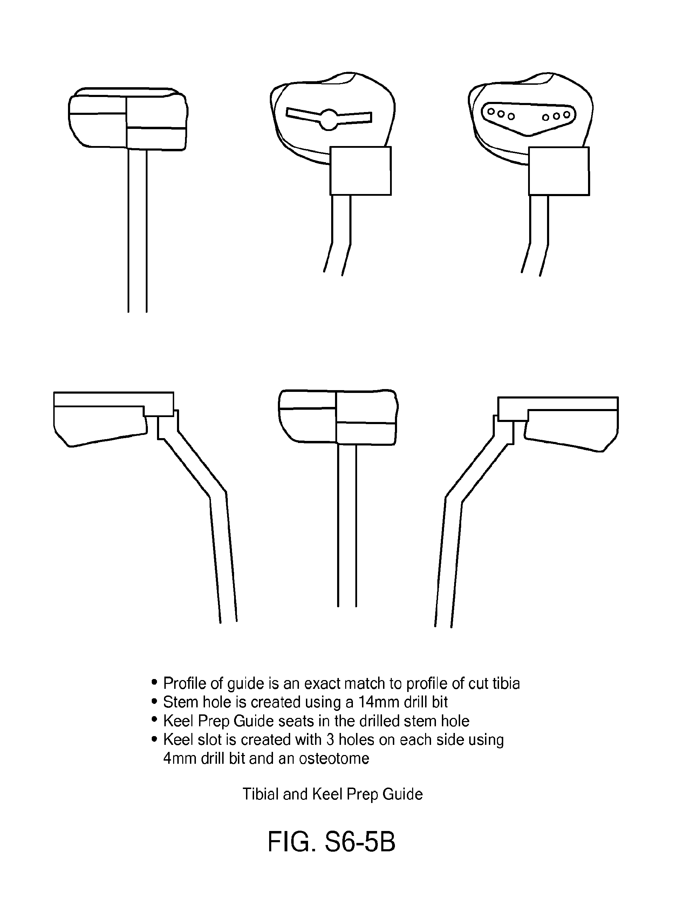

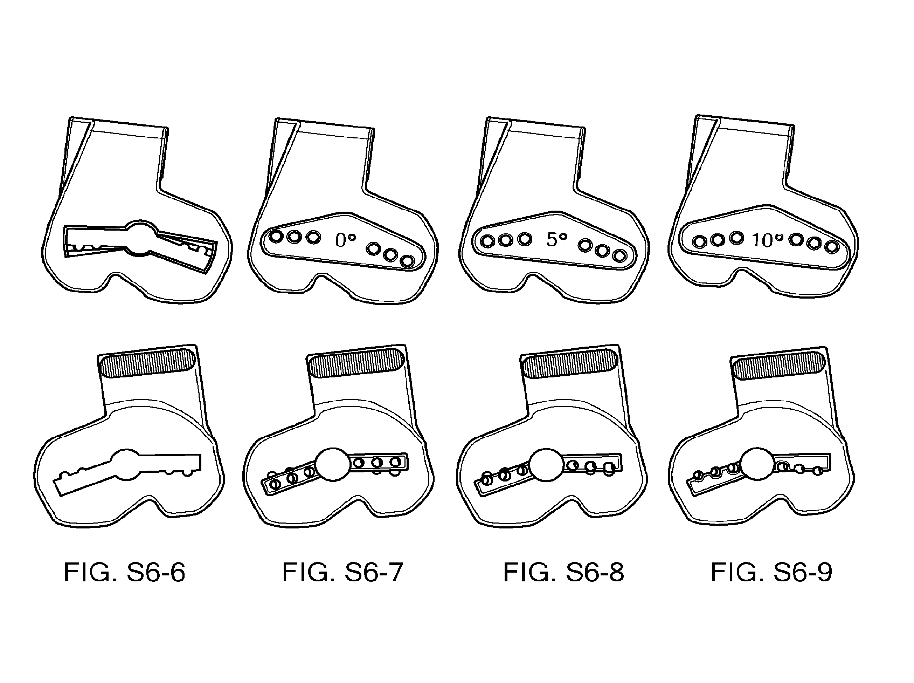

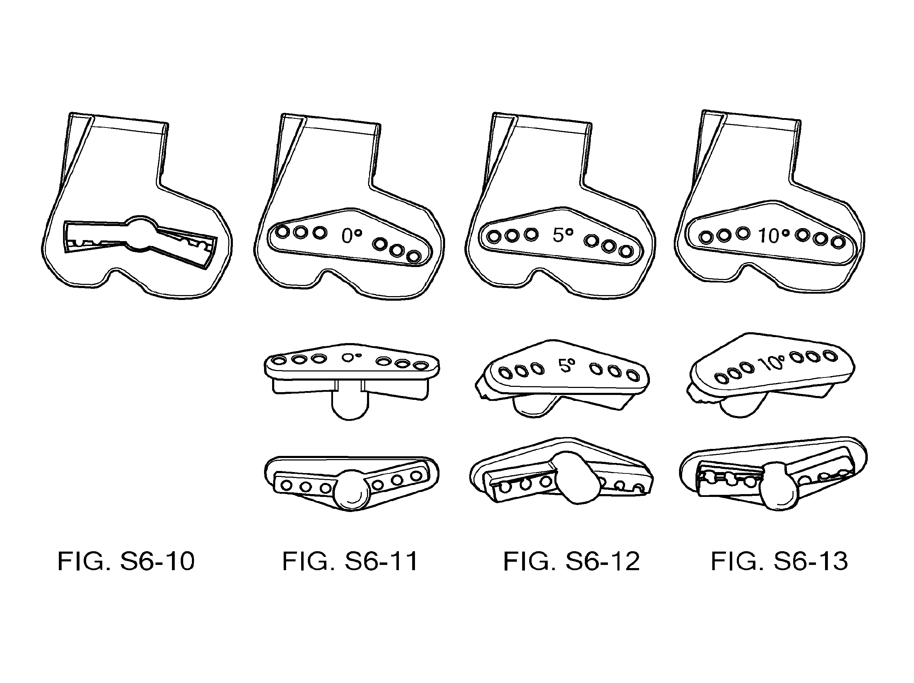

FIGS. S6-2E to S6-2H show another embodiment of a single guide tool and attachments that can be used to establish the peg holes and all the resection cuts associated with installation of a femoral implant component. In particular, the guide tool shown in FIGS. 6-2E includes three reinforcement bars between the implant component condyles. This guide tool can be presented in one or more thicknesses. As noted above, a guide tool having an appropriate thickness can be used to balance the knee before the surgeon performs any femoral cutting. FIG. S6-2F shows the guide tool of FIG. S6-2E with an additional attachment to supply additional surface area to guide to a drilling tool in making the two peg holes. FIG. S6-2G shows the guide tool of FIG. S6-2E with an additional attachment to supply additional surface area to guide a cutting tool in making the numbered resection cuts. As shown in the figure, the attachment covers the entire surface area of the guide tool. The attachment also include flanges adjacent to each cutting hole that supply additional surface area for guiding the cutting tool. FIG. S6-2H shows the reverse side of the guide tool and attachment shown in FIG. S6-2G. As shown, the attachment can be fixed in place on the guide tool using an interface that can include fitting a peg from the attachment into one or both peg guide holes of the guide tool.

The resection cut slots in a particular guide tool can allow for a complete resection cut or a partial resection cut, e.g., scoring of the patient's bone to establish a cut that can be finished after removing the tool. This approach can be advantageous by allowing for faster cutting in the absence of the guide tool. Moreover, one or more resection cut slots can include a blade-depth stop. This is particularly useful for vertical step cuts connecting two facets of a cut.

### 3.4 Guide tool markings

In certain embodiments, one or more guide tools described herein can include markings to identify relevant features, for example, alignment indicators for anatomical and/or biomechanical axes. Such markings can intraoperatively guide a surgeon in the installation procedure. For example, the guide tool shown in FIG. S6-2 includes on its surface alignment indicators for the patient's Whiteside's AP trochlear line, the transepicondylar axis (TEA), and the posterior condylar axis (PEA). These indicators can be in colors and/or in raised geometries to strengthen the guide tool.

Moreover, as shown in FIG. S6-2C, resection cut apertures on a guide tool can include numbers or other instructions to direct a surgeon in the proper resection procedure.

### 3.5 Tibial guide tool

Guide tools for establishing tibial resection cuts can include one or more patient-specific features and/or one or more patient-engineered features, and/or one or more standard features. For example, the bone-facing surface of a tibial resection guide tool can include patient specific features that conform to the uncut tibial surface, e.g., bone and/or cartilage surface. Embodiments of patient-specific tibial resection guide tools are shown in FIGS. S6-4A and S6-4B, and in FIGS. S6-5A, and S6-5B.

FIGS. S6-4A and S6-4B show a tibial guide tool, a tibial guide rod, and a tibial + 2mm additional resection cut guide. The tibial guide tool having a large anterior portion provide a patient-specific surface area that matches a portion of the contour of the tibia. The anterior portion also is relatively thin to make the tool low profile. As shown in the figures, the guide tool includes parallel pins for ease of removal and installation, and a cutting slot that extends laterally (e.g., 10 mm laterally) and is 1.32 mm in diameter to better control the blade flex during cutting. In certain embodiments, the tibial guide tool includes a large medial surface that can be patient-specific for a portion of the patient's corresponding biological structure (e.g., bone or cartilage surface). In this way, the conforming guide tool fits optimally on the particular patient's bone and thereby provides a secure cutting surface. In certain embodiments, the tibial guide tool can include a feature for attaching a tibial guide rod, for example, at the anterior portion of the guide tool. In particular, the tibial guide rod can mate with the anterior feature of the tibial guide via a rectangular boss and hole. As with the guide tool, the guide rod can include one or more patient-specific features and/or one or more patient-engineered features and/or one or more standard features. For the example, the diameter and/or length of the guide rod can be designed based on the specific patient information, such as information derived from one or more patient images. The guide rod depicted in the figure includes a diameter of 8.5 mm and a length of 250 mm.

As shown in FIG. S6-4B, one or more additional guide tools can be included and used to remove additional bone from the proximal surface of the tibia. For example, as shown in the figure, an additional guide tool can provide a 2 mm deeper resection than is available with a first guide tool. Alternatively, one or more additional guide tools can allow for resection of an additional 1 mm, 3 mm, 4 mm, and/or 5 mm (or any amount in between these values) off the patient's proximal tibia. A single additional guide tool can include one or more resection slots to allow for more than one additional resection depth to be achieved with that single guide tool. The one or more additional guide tools can include one or more patient-specific features, one or more patient-engineered features, and one or more standard features.

FIG. S6-5A shows a tibial guide tool for making the same resection cut as shown for FIG. S6-4A. However, the guide tool shown in FIG. S6-5A includes additional patient-adapted surface area that conforms to the patient's biological surface. This patient-adapted surface area can be designed to conform with the patient's biological structure, e.g., subchondral bone and/or cartilage surface, based on one or more images of the patient's joint. Alternatively, this conforming surface area can be engineered based on patient-specific images to conform to an expanded outline of the patient's biological structure, e.g., the patient's subchondral bone surface expanded to include estimated amount of cartilage, for example, expanded 1 mm, 1.5 mm, or 2 mm. For example, the guide tool shown in FIG. S6-5A can be used to make a 2 mm cut at a 5 degree A-P slope. The guide rod can be as described above.

FIG. S6-5B shows a tibial guide tool to resect into the cut tibial surface to create a notch for accepting the keel of a tibial implant component. In certain embodiments, the perimeter of this tool can be patient-specific to match the perimeter of the patient's cut tibial surface. In certain embodiments, the perimeter of this tool can match the perimeter of the tibial implant component that will rest on the cut tibial surface. Alternatively, the perimeter of this tool can be engineered to be some amount less than the perimeter of the patient's cut tibia. The amount that the perimeter is less that the cut tibia can be, for example, a percentage of surface area or circumferential distance, for example, 2% less, 4% less, 5% less, 6% less, 8% less, 10% less, or some other percent less than the corresponding measure on the patient's cut tibia. Alternatively, the tibial guide tool and also, optionally, the tibial implant component, can include a perimeter that is less than the corresponding measure on the patient's cut tibia by an amount calculated to allow for a certain level of intraoperative rotation of the tibial implant component without any overhang of the tibial implant component perimeter.

Various methods can be used to allow for intraoperative rotation of the tibial implant component during installation of the implant. Being able to rotate and install a rotated tibial implant component can be important in balancing the joint with the implant. One example of a method for intraoperatively preparing for a rotated tibial implant component is shown in FIGS. S6-6 through S6-9. In particular, FIGS. S6-6 through S6-9 show the front and back views of the same tibial guide tool to resect into the cut tibial surface to create a notch for accepting the keel of a tibial implant component. FIGS. S6-7 through S6-9 each additionally show an insert with holes for guiding a drilling tool. The holes in each figure are oriented at different angles. Specifically, the insert in FIG. S6-7 can create holes at a 0 degree rotation angle; the insert in FIG. S6-8 can create holes at a 5 degree rotation angle; and the insert in FIG. S6-9 can create holes at a 5 degree rotation angle. Accordingly, by supplying along with the guide tool a series of inserts with holes at various angles, the resected holes and subsequent notch can be rotated at any desired angle (for which an insert has been supplied), which in turn will rotate the tibial implant component by the same angle as its keel is placed into the notch. Inserts can be included to have any degree of rotated holes, for example, inserts can be included to rotate the notch and implant 1, 2, 3, 4, 5, 6, 7, 8, 9, 10 degrees or more. Any number of inserts with different hole rotations can be included. In certain embodiments, the a maximum rotation for a given tibial implant component can be calculated as the rotation at which an undersized component perimeter extends beyond (i.e., overhangs) the perimeter of the patient's cut tibial surface, and inserts can be limited to this maximum rotation. FIGS. S6-10 through S6-13 show the same front view of the guide tools and inserts shown in FIGS. S6-6 through S6-9 and, in addition, show front and back view of three exemplary inserts. As shown in the figures, in certain embodiments the inserts can include differently keyed insert shapes.

### 4. Manufacturing

Implant components can be generated and adapted to a patient's anatomy using any technique known in the art today and in the future. Such techniques include, but are not limited to additive as well as subtractive methods, i.e., methods that add material, for example to a standard blank, and methods that remove material, for example from a standard blank.

Various technologies appropriate for this purpose are known in the art, for example, as described in Wohlers Report 2009, State of the Industry Annual Worldwide Progress Report on Additive Manufacturing, Wohlers Associates, 2009 (ISBN 0-9754429-5-3), available from the web www.wohlersassociates.com; Pham and Dimov, Rapid manufacturing, Springer-Verlag, 2001 (ISBN 1-85233-360-X); Grenda, Printing the Future, The 3D Printing and Rapid Prototyping Source Book, Castle Island Co., 2009; Virtual Prototyping & Bio Manufacturing in Medical Applications, Bidanda and Bartolo (Eds.), Springer, December 17, 2007 (ISBN: 10: 0387334297; 13: 978-0387334295); Bio-Materials and Prototyping Applications in Medicine, Bártolo and Bidanda (Eds.), Springer, December 10, 2007 (ISBN: 10: 0387476822; 13: 978-0387476827); Liou, Rapid Prototyping and Engineering Applications: A Toolbox for Prototype Development, CRC, September 26, 2007 (ISBN: 10: 0849334098; 13: 978-0849334092); Advanced Manufacturing Technology for Medical Applications: Reverse Engineering, Software Conversion and Rapid Prototyping, Gibson (Ed.), Wiley, Jan. 2006 (ISBN: 10: 0470016884; 13: 978-0470016886); and Branner et al., "Coupled Field Simulation in Additive Layer Manufacturing," 3rd International Conference PMI, 2008 (10 pages).

Exemplary techniques for adapting an implant to a patient's anatomy include, but are not limited to those shown in **Table 7.**

**Table 7: Exemplary techniques for forming or altering a patient-specific and/or patient-engineered implant component for a patient's anatomy**

| **Technique** | **Brief description of technique and related notes** |
|---|---|
| **CNC** | CNC refers to computer numerically controlled (CNC) machine tools, a computer-driven technique, e.g., computer-code instructions, in which machine tools are driven by one or more computers. Embodiments of this method can interface with CAD software to streamline the automated design and manufacturing process. |
| **CAM** | CAM refers to computer-aided manufacturing (CAM) and can be used to describe the use of software programming tools to efficiently manage manufacturing and production of products and prototypes. CAM can be used with CAD to generate CNC code for manufacturing three-dimensional objects. |
| **Casting, including casting using rapid prototyped casting patterns** | Casting is a manufacturing technique that employs a mold. Typically, a mold includes the negative of the desired shape of a product. A liquid material is poured into the mold and allowed to cure, for example, with time, cooling, and/or with the addition of a solidifying agent. The resulting solid material or casting can be worked subsequently, for example, by sanding or bonding to another casting to generate a final product. |
| **Welding** | Welding is a manufacturing technique in which two components are fused together at one or more locations. In certain embodiments, the component joining surfaces include metal or thermoplastic and heat is administered as part of the fusion technique. |
| **Forging** | Forging is a manufacturing technique in which a product or component, typically a metal, is shaped, typically by heating and applying force. |
| **Rapid prototyping** | Rapid prototyping refers generally to automated construction of a prototype or product, typically using an additive manufacturing technology, such as EBM, SLS, SLM, SLA, DMLS, 3DP, FDM and other technologies |
| **EBM®** | EBM®) refers to electron beam melting (EBM®), which is a powder-based additive manufacturing technology. Typically, successive layers of metal powder are deposited and melted with an electron beam in a vacuum. |
| **SLS** | SLS refers to selective laser sintering (SLS), which is a powder-based additive manufacturing technology. Typically, successive layers of a powder (e.g., polymer, metal, sand, or other material) are deposited and melted with a scanning laser, for example, a carbon dioxide laser. |
| **SLM** | SLM refers to selective laser melting™ (SLM), which is a technology similar to SLS; however, with SLM the powder material is fully melted to form a fully-dense product. |
| **SLA or SL** | SLA or SL refers to stereolithography (SLA or SL), which is a liquid-based additive manufacturing technology. Typically, successive layers of a liquid resin are exposed to a curing, for example, with UV laser light, to solidify each layer and bond it to the layer below. This technology typically requires the additional and removal of support structures when creating particular geometries. |
| **DMLS** | DMLS refers to direct metal laser sintering (DMLS), which is a powder-based additive manufacturing technology. Typically, metal powder is deposited and melted locally using a fiber optic laser. Complex and highly accurate geometries can be produced with this technology. This technology supports net-shaping, which means that the product generated from the technology requires little or no subsequent surface finishing. |
| **LC** | LC refers to LaserCusing®(LC), which is a powder-based additive manufacturing technology. LC is similar to DMLS; however, with LC a high-energy laser is used to completely melt the powder, thereby creating a fully-dense product. |
| **3DP** | 3DP refers to three-dimensional printing (3DP), which is a highspeed additive manufacturing technology that can deposit various types of materials in powder, liquid, or granular form in a printer-like fashion. Deposited layers can be cured layer by layer or, alternatively, for granular deposition, an intervening adhesive step can be used to secure layered granules together in bed of granules and the multiple layers subsequently can be cured together, for example, with laser or light curing. |
| **LENS** | LENS® refers to Laser Engineered Net Shaping™ (LENS®), which is powder-based additive manufacturing technology. Typically, a metal powder is supplied to the focus of the laser beam at a deposition head. The laser beam melts the powder as it is applied, in raster fashion. The process continues layer by and layer and requires no subsequent curing. This technology supports net-shaping, which means that the product generated from the technology requires little or no subsequent surface finishing. |
| **FDM** | FDM refers to fused deposition modeling™ (FDM) is an extrusion-based additive manufacturing technology. Typically, beads of heated extruded polymers are deposited row by row and layer by layer. The beads harden as the extruded polymer cools. |

### 4.1 Implant components generated from different manufacturing methods

Implant components generated by different techniques can be assessed and compared for their accuracy of shape relative to the intended shape design, for their mechanical strength, and for other factors. In this way, different manufacturing techniques can supply another consideration for achieving an implant component design with one or more target features. For example, if accuracy of shape relative to the intended shape design is critical to a particular patient's implant component design, then the manufacturing technique supplying the most accurate shape can be selected. If a minimum implant thickness is critical to a particular patient's implant component design, then the manufacturing technique supplying the highest mechanical strength and therefore allowing the most minimal implant component thickness, can be selected. Branner *et al.* describe a method a method for the design and optimization of additive layer manufacturing through a numerical coupled-field simulation, based on the finite element analysis (FEA). Branner's method can be used for assessing and comparing product mechanical strength generated by different additive layer manufacturing techniques, for example, SLM, DMLS, and LC.

In certain embodiments, an implant can include components and/or implant component parts produced via various methods. For example, in certain embodiments for a knee implant, the knee implant can include a metal femoral implant component produced by casting or by an additive manufacturing technique and having a patient-specific femoral intercondylar distance; a tibial component cut from a blank and machined to be patient-specific for the perimeter of the patient's cut tibia; and a tibial insert having a standard lock and a top surface that is patient-specific for at least the patient's intercondylar distance between the tibial insert dishes to accommodate the patient-specific femoral intercondylar distance of the femoral implant.

As another example, in certain embodiments a knee implant can include a metal femoral implant component produced by casting or by an additive manufacturing technique that is patient-specific with respect to a particular patient's M-L dimension and standard with respect to the patient's femoral intercondylar distance; a tibial component cut from a blank and machined to be patient-specific for the perimeter of the patient's cut tibia; and a tibial insert having a standard lock and a top surface that includes a standard intercondylar distance between the tibial insert dishes to accommodate the standard femoral intercondylar distance of the femoral implant.

### 4.2 Repair Materials

A wide variety of materials find use in the practice of the present invention, including, but not limited to, plastics, metals, crystal free metals, ceramics, biological materials (e.g., collagen or other extracellular matrix materials), hydroxyapatite, cells (e.g., stem cells, chondrocyte cells or the like), or combinations thereof. Based on the information (e.g., measurements) obtained regarding the defect and the articular surface and/or the subchondral bone, a repair material can be formed or selected. Further, using one or more of these techniques described herein, a cartilage replacement or regenerating material having a curvature that will fit into a particular cartilage defect, will follow the contour and shape of the articular surface, and will match the thickness of the surrounding cartilage. The repair material can include any combination of materials, and typically includes at least one non-pliable material, for example materials that are not easily bent or changed.

Currently, joint repair systems often employ metal and/or polymeric materials including, for example, prostheses which are anchored into the underlying bone (e.g., a femur in the case of a knee prosthesis). See, e.g., U.S. Pat. No. 6,203,576 to Afriat et al. issued Mar. 20, 2001 and U.S. Pat. No. 6,322,588 to Ogle, et al. issued Nov. 27, 2001, and references cited therein. A wide-variety of metals is useful in the practice of the present invention, and can be selected based on any criteria. For example, material selection can be based on resiliency to impart a desired degree of rigidity. Non-limiting examples of suitable metals include silver, gold, platinum, palladium, iridium, copper, tin, lead, antimony, bismuth, zinc, titanium, cobalt, stainless steel, nickel, iron alloys, cobalt alloys, such as Elgiloy®, a cobalt-chromium-nickel alloy, and MP35N, a nickel-cobalt-chromiummolybdenum alloy, and Nitinol T™, a nickel-titanium alloy, aluminum, manganese, iron, tantalum, crystal free metals, such as Liquidmetal® alloys (available from LiquidMetal Technologies, www.liquidmetal.com), other metals that can slowly form polyvalent metal ions, for example to inhibit calcification of implanted substrates in contact with a patient's bodily fluids or tissues, and combinations thereof.

Suitable synthetic polymers include, without limitation, polyamides (e.g., nylon), polyesters, polystyrenes, polyacrylates, vinyl polymers (e.g., polyethylene, polytetrafluoroethylene, polypropylene and polyvinyl chloride), polycarbonates, polyurethanes, poly dimethyl siloxanes, cellulose acetates, polymethyl methacrylates, polyether ether ketones, ethylene vinyl acetates, polysulfones, nitrocelluloses, similar copolymers and mixtures thereof. Bioresorbable synthetic polymers can also be used such as dextran, hydroxyethyl starch, derivatives of gelatin, polyvinylpyrrolidone, polyvinyl alcohol, poly[N-(2-hydroxypropyl) methacrylamide], poly(hydroxy acids), poly(epsilon-caprolactone), polylactic acid, polyglycolic acid, poly(dimethyl glycolic acid), poly(hydroxy butyrate), and similar copolymers.

Other appropriate materials include, for example, the polyketone known as polyetheretherketone (PEEKT). This includes the material PEEK 450G, which is an unfilled PEEK approved for medical implantation available from Victrex of Lancashire, Great Britain. (Victrex is located at www.matweb.com or see Boedeker www.boedeker.com). Other sources of this material include Gharda located in Panoli, India (www.ghardapolymers.com).

It should be noted that the material selected can also be filled. For example, other grades of PEEK are also available and contemplated, such as 30% glass-filled or 30%carbon filled, provided such materials are cleared for use in implantable devices by the FDA, or other regulatory body. Glass filled PEEK reduces the expansion rate and increases the flexural modulus of PEEK relative to that portion which is unfilled. The resulting product is known to be ideal for improved strength, stiffness, or stability. Carbon filled PEEK is known to enhance the compressive strength and stiffness of PEEK and lower its expansion rate. Carbon filled PEEK offers wear resistance and load carrying capability.

As will be appreciated, other suitable similarly biocompatible thermoplastic or thermoplastic polycondensate materials that resist fatigue, have good memory, are flexible, are deflectable, have very low moisture absorption, and/or have good wear and/or abrasion resistance, can be used without departing from the scope of the invention. The implant can also be comprised of polyctherketoneketone (PEKK).

Other materials that can be used include polyetherketone (PEK), polyetherketoneetherketoneketone (PEKEKK), and polyetheretherketoneketone (PEEKK), and, generally, a polyaryletheretherketone. Further, other polyketones can be used as well as other thermoplastics.

Reference to appropriate polymers that can be used for the implant can be made to the following documents, all of which are incorporated herein by reference. These documents include: PCT Publication WO 02/02158 A1, dated Jan. 10, 2002 and entitled Bio-Compatible Polymeric Materials; PCT Publication WO 02/00275 A1, dated Jan. 3, 2002 and entitled Bio-Compatible Polymeric Materials; and PCT Publication WO 02/00270 A1, dated Jan. 3, 2002 and entitled Bio-Compatible Polymeric Materials.

The polymers can be prepared by any of a variety of approaches including conventional polymer processing methods. Preferred approaches include, for example, injection molding, which is suitable for the production of polymer components with significant structural features, and rapid prototyping approaches, such as reaction injection molding and stereo-lithography. The substrate can be textured or made porous by either physical abrasion or chemical alteration to facilitate incorporation of the metal coating. Other processes are also appropriate, such as extrusion, injection, compression molding and/or machining techniques. Typically, the polymer is chosen for its physical and mechanical properties and is suitable for carrying and spreading the physical load between the joint surfaces.

More than one metal and/or polymer can be used in combination with each other. For example, one or more metal-containing substrates can be coated with polymers in one or more regions or, alternatively, one or more polymer-containing substrate can be coated in one or more regions with one or more metals.

The system or prosthesis can be porous or porous coated. The porous surface components can be made of various materials including metals, ceramics, and polymers. These surface components can, in turn, be secured by various means to a multitude of structural cores formed of various metals. Suitable porous coatings include, but are not limited to, metal, ceramic, polymeric (e.g., biologically neutral elastomers such as silicone rubber, polyethylene terephthalate and/or combinations thereof or combinations thereof. See, e.g., U.S. Pat. No. 3,605,123 to Hahn, issued Sep. 20,1971. U.S. Pat. No. 3,808,606 to Tronzo issued May 7, 1974 and U.S. Pat. No. 3,843,975 to Tronzo issued Oct. 29, 1974; U.S. Pat. No. 3,314,420 to Smith issued Apr. 18, 1967; U.S. Pat. No. 3,987,499 to Scharbach issued Oct. 26, 1976; and German Offenlegungsschrift 2,306,552. There can be more than one coating layer and the layers can have the same or different porosities. See, e.g., U.S. Pat. No. 3,938,198 to Kahn, et al., issued Feb. 17, 1976.

The coating can be applied by surrounding a core with powdered polymer and heating until cured to form a coating with an internal network of interconnected pores. The tortuosity of the pores (e.g., a measure of length to diameter of the paths through the pores) can be important in evaluating the probable success of such a coating in use on a prosthetic device. See, also, U.S. Pat. No. 4,213,816 to Morris issued Jul. 22, 1980. The porous coating can be applied in the form of a powder and the article as a whole subjected to an elevated temperature that bonds the powder to the substrate. Selection of suitable polymers and/or powder coatings can be determined in view of the teachings and references cited herein, for example based on the melt index of each.

## Claims

1. A patient-specific femoral knee implant for implantation on a portion of a femur of a patient's knee, comprising:
a first condylar portion having a first bone-facing surface for abutting at least a portion of a first condyle of the patient's knee and a first articular surface generally opposite the first bone-facing surface;
the first articular surface having a first patient-specific curvature generally disposed in a first plane, the first patient-specific curvature being a predetermined curvature based on the dimensions of the knee to substantially replicate a corresponding curvature of the patient's first condyle when the first bone-facing surface abuts the first condyle; said corresponding curvature preferably being a J-curve;
the first articular surface having a standard curvature in a second plane.

2. The implant of claim 1, wherein the patient-specific curvature extends along a portion of the length of the first articular surface, preferably wherein the patient-specific curvature extends along the entire length of a weight bearing area of the implant, or along a portion of the length of a weight bearing area of the implant.

3. The implant of claim 1, wherein the first plane is a sagittal plane, or a coronal plane.

4. The implant of claim 1, wherein the patient-specific curvature is a smoothed curvature that eliminates or reduces at least some local maxima of a curvature of the patient's condyle.

5. The implant of claim 1, wherein the standard curvature extends along substantially the entire length of the articular surface, or along a portion of the length of the articular surface, preferably wherein the standard curvature extends along the entire length of a weight bearing area of the implant or along a portion of the length of a weight bearing area of the implant.

6. The implant of claim 1, wherein the standard curvature extends along substantially the entire length of the articular surface generally in the direction of the first plane or along a portion of the length of the articular surface generally in the direction of the first plane, preferably wherein the standard curvature extends along a portion of the length of a weight bearing area of the implant generally in the direction of the first plane.

7. The implant of claim 1, wherein the second plane is a sagittal plane or a coronal plane.

8. The implant of claim 1, wherein the standard curvature approximates an average curvature of a corresponding curvature of the patient's condyle.

9. The implant of claim 1, further comprising
a second condylar portion having a second bone-facing surface for abutting at least a portion of a second condyle of the patient's knee and a second articular surface generally opposite the second bone-facing surface;
the second articular surface having a second patient-specific curvature generally disposed in a third plane, the second patient-specific curvature being a predetermined curvature based on the dimensions of the patient's knee to substantially replicate a corresponding curvature of the patient's second condyle when the second bone-facing surface abuts the second condyle.

10. The implant of claim 9, wherein the first and third planes are generally sagittal planes, or substantially coronal planes.

11. The implant of claim 9, wherein the first patient specific curvature extends for substantially the entire length of a weight-bearing portion of the first articular surface, or for a portion of a weight-bearing portion of the first articular surface, or wherein the second patient-specific curvature extends for substantially the entire length of a weight-bearing portion of the second articular surface, or for a portion of the length of a weight-bearing portion of the second articular surface, or for most of the length of a weight-bearing portion of the second articular surface.

12. The implant of claim 1 or 9, further comprising one or more additional implant features or measurements derived from patient-specific data and adapted for the particular patient.

13. The implant of claim 12, wherein the one or more additional features or measurements includes one or more of
a) one or more planar facets on the bone- facing surface of the femoral implant being derived from patient-specific data and adapted to maximize bone preservation for the particular patient;
b) a condylar width of the implant being derived from patient-specific data and adapted to substantially match a corresponding width of the patient's femoral condyle, or a predetermined percentage thereof;
c) a distance between medial and lateral condyles on the implant being derived from patient-specific data and adapted to substantially match a corresponding distance between the patient's medial and lateral femoral condyles, or a predetermined percentage thereof;
d) a thickness of the implant from the bone-facing surface to the articular surface being derived from patient-specific data and adapted to substantially match a corresponding thickness from a corresponding planned resection cut surface to a corresponding articular surface on the patient's femur, or a predetermined percentage thereof;
e) a cross-sectional perimeter shape of the implant being derived from patient-specific data and adapted to substantially match a corresponding cross-sectional perimeter shape of the patient's femur, or a predetermined percentage thereof;
f) a volume of a portion of the implant derived from patient-specific data and adapted to substantially match a corresponding volume of a portion of the patient's femur, or a predetermined percentage thereof.

## Patentansprüche

1. Ein patientenspezifisches femorales Knieimplantat zur Implantation auf einen Teil eines Femurs eines Knies eines Patienten, umfassend:
einen ersten Kondylenteil mit einer ersten auf den Knochen gerichteten Oberfläche zum Aneinandergrenzen an mindestens einen Teil einer ersten Kondyle des Knies des Patienten und einer ersten artikulären Oberfläche, die im Allgemeinen gegenüber der ersten auf den Knochen gerichteten Oberfläche ist;
wobei die erste artikuläre Oberfläche eine erste patientenspezifische Krümmung hat, die im Allgemeinen in einer ersten Ebene angeordnet ist, wobei die erste patientenspezifische Krümmung eine vorbestimmte Krümmung basierend auf den Dimensionen des Kies ist, um im Wesentlichen eine entsprechende Krümmung der ersten Kondyle des Patienten nachzubilden, wenn die erste auf den Knochen gerichtete Oberfläche an die erste Kondyle angrenzt; wobei jene entsprechende Krümmung vorzugsweise eine J-Kurve ist;
wobei die erste artikuläre Oberfläche eine Standardkrümmung in einer zweiten Ebene hat.

2. Das Implantat nach Anspruch 1, wobei sich die patientenspezifische Krümmung entlang eines Teils der Länge der ersten artikulären Oberfläche erstreckt, wobei sich die patientenspezifische Krümmung vorzugsweise entlang der gesamten Länge eines belasteten Bereichs des Implantats oder entlang eines Teils der Länge eines belasteten Bereichs des Implantats erstreckt.

3. Das Implantat nach Anspruch 1, wobei die erste Ebene eine Sagittalebene oder Koronalebene ist.

4. Das Implantat nach Anspruch 1, wobei die patientenspezifische Krümmung eine geglättete Krümmung ist, die mindestens einige lokale Maxima einer Krümmung der Kondyle des Patienten beseitigt oder verringert.

5. Das Implantat nach Anspruch 1, wobei sich die Standardkrümmung entlang der im Wesentlichen gesamten Länge der artikulären Oberfläche , oder entlang eines Teils der Länge der artikulären Oberfläche erstreckt, wobei sich die Standardkrümmung vorzugsweise entlang der gesamten Länge eines belasteten Bereichs des Implantats oder entlang eines Teils der Länge eines belasteten Bereichs des Implantats erstreckt.

6. Das Implantat nach Anspruch 1, wobei sich die Standardkrümmung im Allgemeinen in Richtung der ersten Ebene entlang der im Wesentlichen gesamten Länge der artikulären Oberfläche oder im Allgemeinen in Richtung der ersten Ebene entlang eines Teils der Länge der artikulären Oberfläche erstreckt, wobei sich die Standardkrümmung vorzugsweise im Allgemeinen in Richtung der ersten Ebene entlang eines belasteten Bereichs des Implantats erstreckt.

7. Das Implantat nach Anspruch 1, wobei die zweite Ebene eine Sagittalebene oder Koronalebene ist.

8. Das Implantat nach Anspruch 1, wobei sich die Standardkrümmung in etwa einer durchschnittlichen Krümmung einer entsprechenden Krümmung der Kondyle des Patienten annähert.

9. Das Implantat nach Anspruch 1, weiter umfassend
einen zweiten Kondylenteil mit einer zweiten auf den Knochen gerichteten Oberfläche zum Aneinandergrenzen an mindestens einen Teil einer zweiten Kondyle des Knies des Patienten und einer zweiten artikulären Oberfläche, die im Allgemeinen gegenüber der zweiten auf den Knochen gerichteten Oberfläche ist;
wobei die zweite artikuläre Oberfläche eine zweite patientenspezifische Krümmung hat, die im Allgemeinen in einer dritten Ebene angeordnet ist, wobei die zweite patientenspezifische Krümmung eine vorbestimmte Krümmung basierend auf den Dimensionen des Kies des Patienten ist, um im Wesentlichen eine entsprechende Krümmung der zweiten Kondyle des Patienten nachzubilden, wenn die zweite auf den Knochen gerichtete Oberfläche an die zweiten Kondyle angrenzt.

10. Das Implantat von Anspruch 9, wobei die erste und dritte Ebene im Allgemeinen Sagittalebenen oder im Wesentlichen Koronalebenen sind.

11. Das Implantat nach Anspruch 9, wobei sich die erste patientenspezifische Krümmung über die im Wesentlichen gesamte Länge eines belasteten Teils der ersten artikulären Oberfläche oder über einen Teil eines belasteten Teils der ersten artikulären Oberfläche erstreckt, oder wobei sich die zweite patientenspezifische Krümmung über die im Wesentlichen gesamte Länge eines belasteten Teils der zweiten artikulären Oberfläche, oder über einen Teil der Länge eines belasteten Teils der zweiten artikulären Oberfläche oder über den Großteil der Länge eines belasteten Teils der zweiten artikulären Oberfläche erstreckt.

12. Das Implantat nach Anspruch 1 oder 9, weiter umfassend ein oder mehr zusätzliche Implantatmerkmale oder -maße, die von patientenspezifischen Daten abgeleitet und für den jeweiligen Patienten angepasst sind.

13. Das Implantat von Anspruch 12, wobei die ein oder mehr zusätzlichen Implantatmerkmale oder -maße ein oder mehr umfassen von
a) ein oder mehr Facetten auf der auf den Knochen gerichteten Oberfläche des femoralen Implantats, die von patientenspezifischen Datan abgeleitet und angepasst sind, um die Knochenerhaltung für den jeweiligen Patienten zu maximieren;
b) eine Kondylenbreite des Implantats, die von patientenspezifischen Daten abgeleitet und angepasst ist, um im Wesentlichen mit einer entprechenden Breite der Femurkondyle des Patienten, oder einem vorbestimmten Prozentsatz davon übereinzustimmen;
c) ein Abstand zwischen medialen und lateralen Kondylen auf dem Implanat, der von patientenspezifischen Daten abgeleitet und angepasst ist, um im Wesentlichen mit einem entsprechenden Abstand zwischen medialen und lateralen femoralen Kondylen des Patienten, oder einem vorbestimmten Prozentsatz davon übereinzustimmen;
d) eine Dicke des Implantats von der auf den Knochen gerichteten Oberfläche bis zur artikulären Oberfläche, die von patientenspezifischen Daten abgeleitet und angepasst ist, um im Wesentlichen mit einer entsprechenden Dicke von einer entsprechenden geebneten Resektionsschnittfläche bis zur entsprechenden artikulären Oberfläche auf dem Femur des Patienten, oder einem vorbestimmten Prozentsatz davon übereinzustimmen;
e) eine Querschnittsumrissform des Implantats, die von patientenspezifischen Daten abgeleitet und angepasst ist, um im Wesentlichen mit einer Querschnittsumrissform des Femurs des Patienten, oder einem vorbestimmten Prozentsatz davon übereinzustimmen;
f) ein Volumen eines Teils des Implantats, abgeleitet von patientenspezifischen Daten und angepasst, um im Wesentlichen mit einem entsprechenden Volumen eines Teils des Femurs des Patienten, oder einem vorbestimmten Prozentsatz davon übereinzustimmen.

## Revendications

1. Implant de genou fémoral spécifique à un patient pour implantation sur une partie d'un fémur d'un genou de patient, comprenant :
une première partie condylienne ayant une première surface orientée vers l'os pour venir en butée contre au moins une partie d'un premier condyle du genou du patient et une première surface articulaire généralement opposée à la première surface orientée vers l'os ;
la première surface articulaire ayant une première courbure spécifique au patient généralement disposée dans un premier plan, la première courbure spécifique au patient étant une courbure prédéterminée basée sur les dimensions du genou pour reproduire sensiblement une courbure correspondante du premier condyle du patient lorsque la première surface orientée vers l'os vient en butée contre le premier condyle ; ladite courbure correspondante étant de préférence une courbe en J ;
la première surface articulaire ayant une courbure standard dans un deuxième plan.

2. Implant selon la revendication 1, dans lequel la courbure spécifique au patient s'étend le long d'une partie de la longueur de la première surface articulaire, de préférence dans lequel la courbure spécifique au patient s'étend le long de toute la longueur d'une zone portante de l'implant, ou le long d'une partie de la longueur d'une zone portante de l'implant.

3. Implant selon la revendication 1, dans lequel le premier plan est un plan sagittal ou un plan frontal.

4. Implant selon la revendication 1, dans lequel la courbure spécifique au patient est une courbure lissée qui élimine ou réduit au moins certains des maxima locaux d'une courbure du condyle du patient.

5. Implant selon la revendication 1, dans lequel la courbure standard s'étend sensiblement sur toute la longueur de la surface articulaire, ou sur une partie de la longueur de la surface articulaire, de préférence dans lequel la courbure standard s'étend le long de toute la longueur d'une zone portante de l'implant ou le long d'une partie de la longueur d'une zone portante de l'implant.

6. Implant selon la revendication 1, dans lequel la courbure standard s'étend sensiblement sur toute la longueur de la surface articulaire, généralement dans la direction du premier plan ou sur une partie de la longueur de la surface articulaire, généralement dans la direction du premier plan, de préférence dans lequel la courbure standard s'étend le long d'une partie de la longueur d'une zone portante de l'implant, généralement dans la direction du premier plan.

7. Implant selon la revendication 1, dans lequel le deuxième plan est un plan sagittal ou un plan frontal.

8. Implant selon la revendication 1, dans lequel la courbure standard se rapproche d'une courbure moyenne d'une courbure correspondante du condyle du patient.

9. Implant selon la revendication 1, comprenant en outre
une seconde partie condylienne ayant une seconde surface orientée vers l'os pour venir en butée contre au moins une partie d'un second condyle du genou du patient et une seconde surface articulaire généralement opposée à la seconde surface orientée vers l'os ;
la seconde surface articulaire ayant une seconde courbure spécifique au patient disposée généralement dans un troisième plan, la seconde courbure spécifique au patient étant une courbure prédéterminée basée sur les dimensions du genou du patient pour reproduire sensiblement une courbure correspondante du second condyle du patient lorsque la seconde surface orientée vers l'os vient en butée contre le second condyle.

10. Implant selon la revendication 9, dans lequel les premier et troisième plans sont généralement des plans sagittaux, ou sensiblement des plans frontaux.

11. Implant selon la revendication 9, dans lequel la première courbure spécifique au patient s'étend sur sensiblement toute la longueur d'une partie portante de la première surface articulaire, ou sur une partie d'une partie portante de la première surface articulaire, ou dans lequel la seconde courbure spécifique au patient s'étend sur sensiblement toute la longueur d'une partie portante de la seconde surface articulaire, ou sur une partie de la longueur d'une partie portante de la seconde surface articulaire, ou sur la majeure partie de la longueur d'une partie portante de la seconde surface articulaire.

12. Implant selon la revendication 1 ou 9, comprenant en outre une ou plusieurs caractéristiques ou mesures d'implant supplémentaires dérivées de données spécifiques au patient et adaptées au patient particulier.

13. Implant selon la revendication 12, dans lequel la ou les caractéristiques ou mesures supplémentaires incluent une ou plusieurs parmi
a) une ou plusieurs facettes planes sur la surface orientée vers l'os de l'implant fémoral dérivées de données spécifiques au patient et adaptées pour optimiser la préservation osseuse pour le patient particulier ;
b) une largeur condylienne de l'implant dérivée de données spécifiques au patient et adaptée pour concorder sensiblement avec une largeur correspondante du condyle fémoral du patient, ou un pourcentage prédéterminé de celle-ci ;
c) une distance entre les condyles médial et latéral sur l'implant dérivée de données spécifiques au patient et adaptée pour concorder sensiblement avec une distance correspondante entre les condyles fémoraux médial et latéral du patient, ou un pourcentage prédéterminée de celle-ci ;
d) une épaisseur de l'implant de la surface orientée vers l'os à la surface articulaire, dérivée de données spécifiques au patient et adaptée pour concorder sensiblement avec une épaisseur correspondante d'une surface de découpe par résection prévue correspondante à une surface articulaire correspondante sur le fémur du patient, ou un pourcentage prédéterminé de celle-ci ;
e) une forme de périmètre en coupe de l'implant dérivée de données spécifiques au patient et adaptée pour concorder sensiblement avec une forme de périmètre en coupe correspondante du fémur du patient, ou un pourcentage prédéterminé de celle-ci ;
f) un volume d'une partie de l'implant dérivé de données spécifiques au patient et adapté pour concorder sensiblement avec un volume correspondant d'une partie du fémur du patient, ou un pourcentage prédéterminé de celui-ci.
